# EUROPEAN PATENT APPLICATION

(11) **EP 4 421 073 A1**
(43) Date of publication of application: **28.08.2024**
(21) Application number: 22882953.7
(22) Date of filing: 20.10.2022
(51) Int. Cl.: C07D 413/12, C07D 403/10, C07D 249/08, A61K 31/41, A61P 9/12, A61P 13/12, A61P 9/10, A61P 3/10

(54) **DUAL ANTAGONIST AND USE THEREOF**

(30) Priority: 21.10.2021 CN 202111228856
(71) Applicant: Nicoya Therapeutics (Shanghai) Co., Ltd, Shanghai 201204 (CN)
(72) Inventor: MA, Ji, Shanghai 201204 (CN); GAO, Yun, Shanghai 201204 (CN); LI, Haiming, Shanghai 201204 (CN); GE, Ping, Shanghai 201204 (CN); MA, Huiyong, Shanghai 201204 (CN); CHEN, Liu, Shanghai 201204 (CN)
(74) Representative: Kuhnen & Wacker Patent- und Rechtsanwaltsbüro PartG mbB
(86) International application number: PCT/CN2022/126482
(87) International publication number: WO 2023/066348

(57) **Abstract**

Provided are a new compound as represented by formula (I) having a dual antagonistic effect of an angiotensin II receptor and an endothelin receptor, and the use thereof in the preparation of a drug.

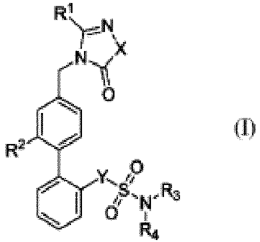

## Description

### CROSS-REFERENCE TO RELATED APPLICATION

The present disclosure claims the priority to and the benefits of Chinese Patent Application No. 202111228856.0, filed on October 21, 2021 and entitled "DUAL ANTAGONIST AND USE THEREOF", the entire contents of which are incorporated herein by reference.

### TECHNICAL FIELD

The present disclosure relates to a class of new compounds having a dual antagonistic effect of an angiotensin II receptor and an endothelin receptor, and use thereof in the manufacture of a medicament.

### BACKGROUND

Angiotensin II (AngII) and endothelin-1 (ET-1) are two potent endogenous vasoactive peptides thought to play roles in controlling both vascular tone and pathological tissue remodeling associated with a variety of diseases. The angiotensin II receptor includes four subtypes, namely, AT1, AT2, AT3, and AT4. Of these, AT1 and AT2 are predominant. The AT1 receptor mediates almost all pathophysiological functions of the angiotensin II receptor. As a new drug against systemic antihypertension, the angiotensin II receptor antagonist has exhibited advantages such as strong affinity, high selectivity, oral availability, long half-life, and good tolerance. Endothelin is a potent vasoactive peptide that promotes vasoconstriction and plays a key role in maintaining vascular homeostasis. The prior arts have identified a variety of different endothelin receptors (e.g. ETA, ETB1, ETB2, and ETC). Among them, ETA is the best-studied receptor, and its antagonists can be used to treat diseases such as hypertension, pulmonary hypertension, chronic renal diseases, and atherosclerosis.

Preclinical and initial clinical data suggest that simultaneous blockade of angiotensin II and endothelin-1 at their respective receptors AT1 and ETA may provide improved treatment options for several cardiovascular diseases, as compared to either of the mechanisms alone. Dual angiotensin II receptor and endothelin receptor antagonists have an antagonistic effect on both the angiotensin II receptor and the ETA receptor, and have a better efficacy and a wider range of application as compared to a single angiotensin II or ETA receptor antagonist, which are a class of drugs potentially usable for the treatment of diseases such as hypertension or the kidney disease. Although patents such as WO2000001389A1 and CN101891735A disclose a series of dual angiotensin II receptor and endothelin receptor antagonists, there still remains a need to develop a dual antagonist drug with better activity, higher selectivity, better water solubility, and fewer drug-drug interactions.

### SUMMARY

The present disclosure provides a compound represented by formula I, or a deuterated compound thereof, or a stereoisomer thereof, or a pharmaceutically acceptable salt thereof: 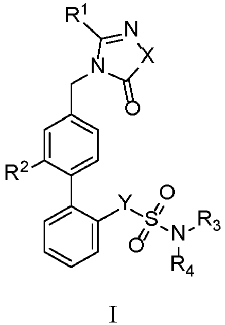 wherein
X is NR^{X1} or CR^{X2}R^{X3};
Y is a chemical bond, NR^{Y1} or CR^{Y2}R^{Y3};
R^{Y1} is selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring);
R^{Y2} and R^{Y3} are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring);
R^{X1} is selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring); wherein the alkylene, carbocyclyl, heterocycloalkyl, aromatic ring, or heteroaromatic ring is optionally further substituted by one, two, three or four independent R^{X11};
each R^{X11} is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR^{X12}, -C₀₋₄ alkylene-SR^{X12}, and -C₀₋₄ alkylene-NR^{X12}R^{X13}; or two independent R^{X11}, together with the atom to which they are linked, form 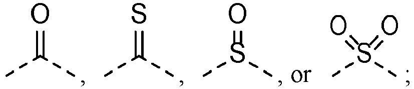
R^{X12} and R^{X13} are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen;
R^{X2} and R^{X3}, together with the atom to which they are linked directly, form a 6- to 12-membered spirocyclic ring, a 6- to 12-membered spiroheterocyclic ring, a 6- to 12-membered fused cyclic ring, or a 6- to 12-membered fused heterocyclic ring; wherein the spirocyclic ring, spiroheterocyclic ring, fused cyclic ring, or fused heterocyclic ring is optionally further substituted by one, two, three or four independent R^{X21};
each R^{X21} is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR^{X22}, -C₀₋₄ alkylene-SR^{X22}, and -C₀₋₄ alkylene-NR^{X22}R^{X23}; or two independent R^{X21}, together with the atom to which they are linked directly, form 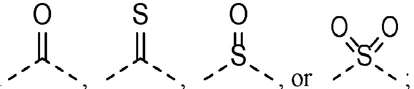
R^{X22} and R^{X23} are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen;
R¹ is selected from the group consisting of -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen;
R² is selected from the group consisting of -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR²¹, -C₀₋₄ alkylene-SR²¹, -C₀₋₄ alkylene-NR²¹R²², -C₀₋₄ alkylene-OC(O)R²¹, -C₀₋₄ alkylene-S(O)₂R²¹, -C₀₋₄ alkylene-S(O)R²¹, -C₀₋₄ alkylene-S(O)₂NR²¹R²², -C₀₋₄ alkylene-S(O)NR²¹R²², -C₀₋₄ alkylene-C(O)R²¹, -C₀₋₄ alkylene-C(O)OR²¹, -C₀₋₄ alkylene-C(O)NR²¹R²², -C₀₋₄ alkylene-NR²¹C(O)R²², -C₀₋₄ alkylene-NR²¹S(O)₂R²², -C₀₋₄ alkylene-NR²¹S(O)R²², -C₀₋₄ alkylene-( 3- to 10-membered carbocyclyl), -C₀₋₄ alkylene-( 4- to 10-membered heterocycloalkyl), -C₀₋₄ alkylene-(6- to 10-membered aromatic ring), and -C₀₋₄ alkylene-(5- to 10-membered heteroaromatic ring);
R²¹ and R²² are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring);
R³ and R⁴ are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring); wherein the alkylene, carbocyclyl, heterocycloalkyl, aromatic ring, or heteroaromatic ring is optionally further substituted by one, two, three or four independent R³¹;
each R³¹ is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR³², -C₀₋₄ alkylene-SR³², -C₀₋₄ alkylene-NR³²R³³, -C₀₋₄ alkylene-OC(O)R³², -C₀₋₄ alkylene-S(O)₂R³², -C₀₋₄ alkylene-S(O)R³², -C₀₋₄ alkylene-S(O)₂NR³²R³³, -C₀₋₄ alkylene-S(O)NR³²R³³, -C₀₋₄ alkylene-C(O)R³², -C₀₋₄ alkylene-C(O)OR³², -C₀₋₄ alkylene-C(O)NR³²R³³, -C₀₋₄ alkylene-NR³²C(O)R³³, -C₀₋₄ alkylene-NR³²S(O)₂R³³, -C₀₋₄ alkylene-NR³²S(O)R³³, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring); or two independent R³¹, together with the atom to which they are linked directly, form 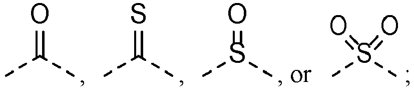
R³² and R³³ are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen.

Further, R¹ is n-butyl.

Further, R² is selected from the group consisting of -C₁₋₂ alkylene-OR²¹, -C₁₋₂ alkylene-SR²¹, -C₁₋₂ alkylene-NR²¹R²², -C₁₋₂ alkylene-OC(O)R²¹, -C₁₋₂ alkylene-S(O)₂R²¹, -C₁₋₂ alkylene-S(O)R²¹, -C₁₋₂ alkylene-S(O)₂NR²¹R²², -C₁₋₂ alkylene-S(O)NR²¹R²², -C₁₋₂ alkylene-C(O)R²¹, -C₁₋₂ alkylene-C(O)OR²¹, -C₁₋₂ alkylene-C(O)NR²¹R²², -C₁₋₂ alkylene-NR²¹C(O)R²², -C₁₋₂ alkylene-NR²¹S(O)₂R²², and -C₁₋₂ alkylene-NR²¹S(O)R²²;
R²¹ and R²² are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₂ alkylene-(3- to 10-membered carbocyclyl), and -C₀₋₂ alkylene-(4- to 10-membered heterocycloalkyl).

Furthermore, R² is selected from the group consisting of 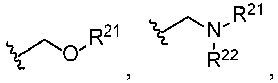 and

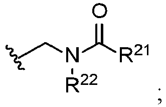

R²¹ and R²² are each independently selected from the group consisting of hydrogen, -C₁₋₆ alkyl, -C₁₋₆ alkyl substituted with halogen, and -C₀₋₂ alkylene-(3-6-membered carbocyclyl).

Further specifically, R² is selected from the group consisting of

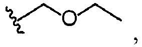

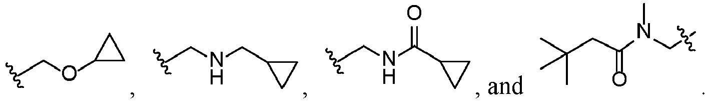

Further, R³ is selected from the group consisting of -C₀₋₂ alkylene-(3- to 10-membered carbocyclyl), -C₀₋₂ alkylene-(4- to 10-membered heterocycloalkyl), -C₀₋₂ alkylene-(6- to 10-membered aromatic ring), and -C₀₋₂ alkylene-(5- to 10-membered heteroaromatic ring); wherein the alkylene, carbocyclyl, heterocycloalkyl, aromatic ring, or heteroaromatic ring is optionally further substituted by one, two, three or four independent R³¹; each R³¹ is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen; or two independent R³¹, together with the atom to which they are linked directly, form 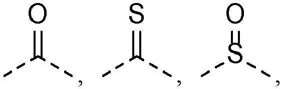 or

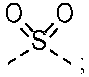

R⁴ is selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen.

Furthermore, R³ is selected from the group consisting of 3-membered carbocyclyl, 4-membered carbocyclyl, 5-membered carbocyclyl, 6-membered carbocyclyl, 7-membered carbocyclyl, 8-membered carbocyclyl, 9-membered carbocyclyl, 10-membered carbocyclyl, 4-membered heterocycloalkyl, 5-membered heterocycloalkyl, 6-membered heterocycloalkyl, 7-membered heterocycloalkyl, 8-membered heterocycloalkyl, 9-membered heterocycloalkyl, 10-membered heterocycloalkyl, 6-membered aromatic ring, 10-membered aromatic ring, 5-membered heteroaromatic ring, 6-membered heteroaromatic ring, 7-membered heteroaromatic ring, 8-membered heteroaromatic ring, 9-membered heteroaromatic ring, and 8-membered heteroaromatic ring; wherein the carbocyclyl, heterocycloalkyl, aromatic ring, or heteroaromatic ring is optionally further substituted by one, two, three or four independent R³¹;

R⁴ is selected from the group consisting of hydrogen and -C₁₋₆ alkyl.

Further specifically, R³ is selected from the group consisting of 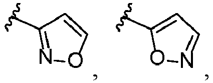 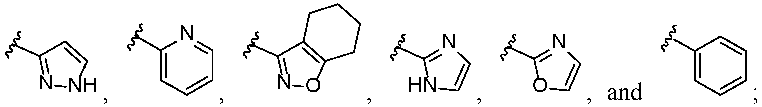 wherein the rings from which R³ is selected is optionally further substituted by one, two, three or four independent R³¹.

Yet further specifically, R³ is selected from the group consisting of 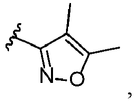 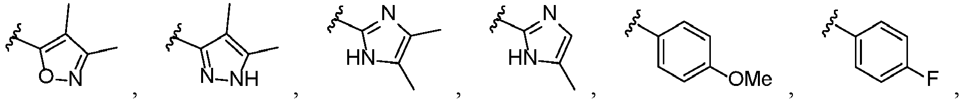 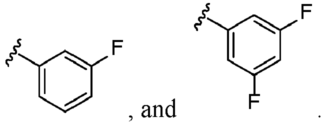 Further, X is NR^{X1};

R^{X1} is selected from the group consisting of -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₂ alkyl-(3- to 10-membered carbocyclyl), -C₀₋₂ alkyl-(4- to 10-membered heterocycloalkyl), -C₀₋₂ alkyl-(6- to 10-membered aromatic ring), and -C₀₋₂ alkyl-(5- to 10-membered heteroaromatic ring).

Furthermore, R^{X1} is selected from the group consisting of -C₁₋₆ alkyl, -C₂₋₆ alkenyl, -C₂₋₆ alkynyl, 3-membered carbocyclyl, 4-membered carbocyclyl, 5-membered carbocyclyl, 6-membered carbocyclyl, 7-membered carbocyclyl, 8-membered carbocyclyl, 4-membered heterocycloalkyl, 5-membered heterocycloalkyl, 6-membered heterocycloalkyl, 7-membered heterocycloalkyl, 8-membered heterocycloalkyl, a benzene ring, a 5-membered heteroaromatic ring, and a 6-membered heteroaromatic ring.

Further specifically, R^{X1} is

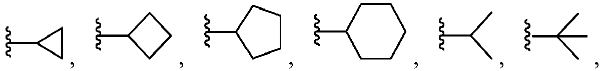

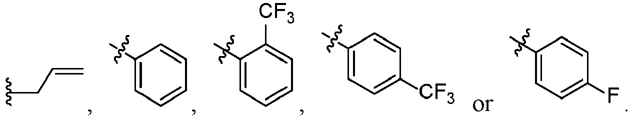

Further, the compound of formula I is represented by formula IIa: 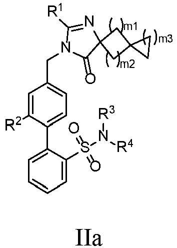 wherein
m1 and m2 are each independently 0, 1, 2 or 3; and
m3 is 1, 2, 3, 4 or 5.

Further, the compound of formula I is represented by formula IIb: 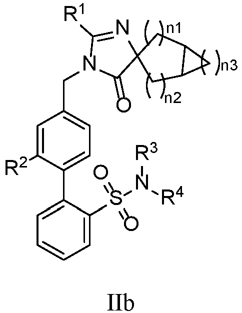 wherein
n1 and n2 are each independently 0, 1, 2 or 3; and
n3 is 1, 2, 3, 4 or 5.

In some specific embodiments of the present disclosure, the compound is:

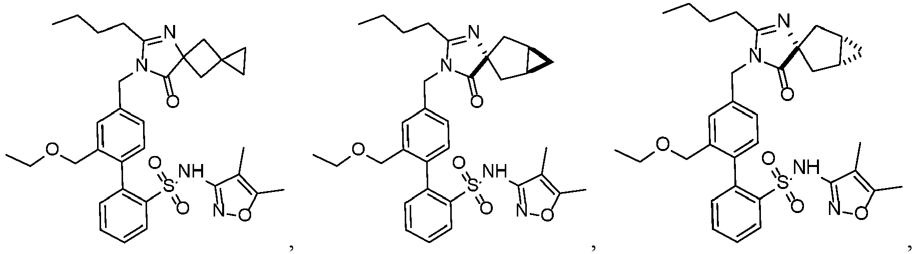

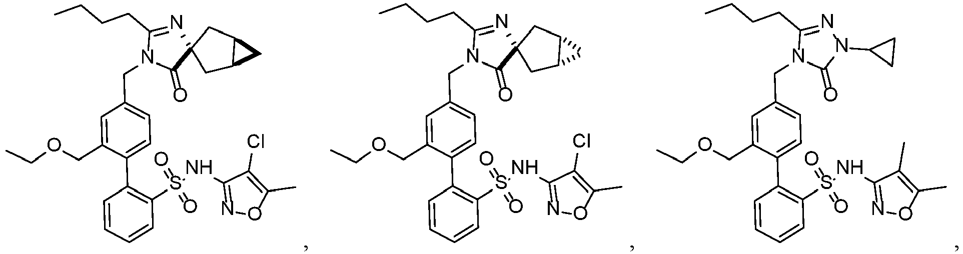

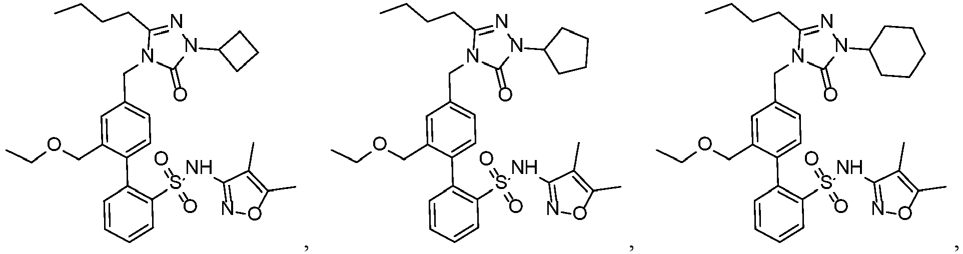

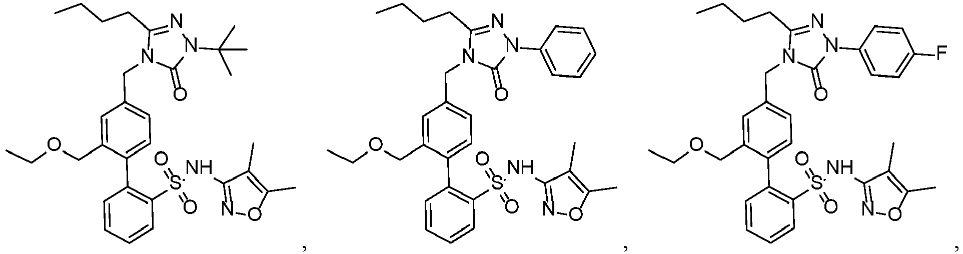

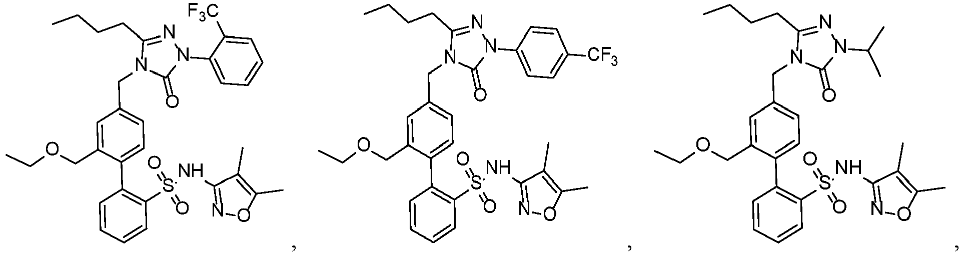

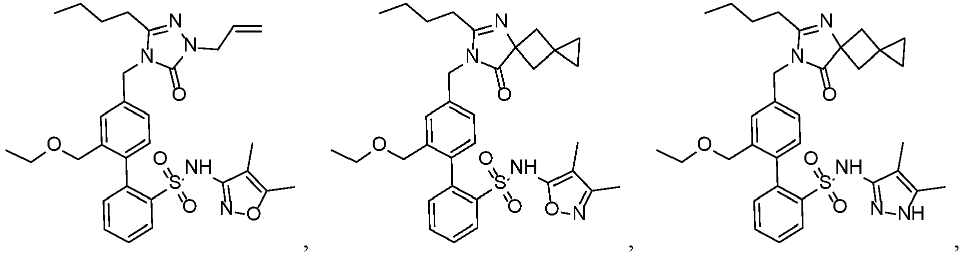

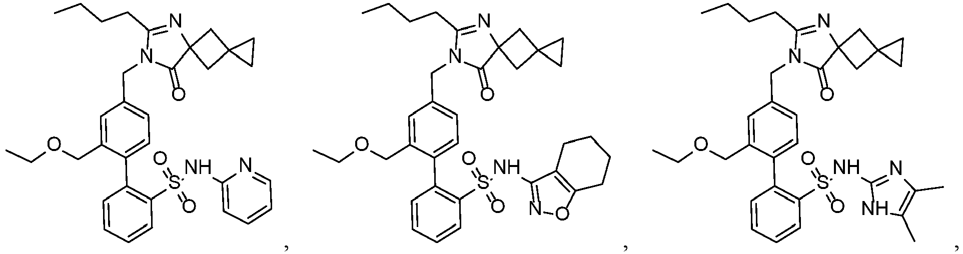

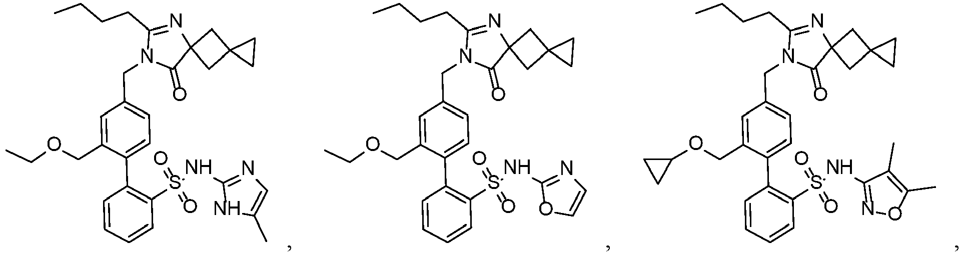

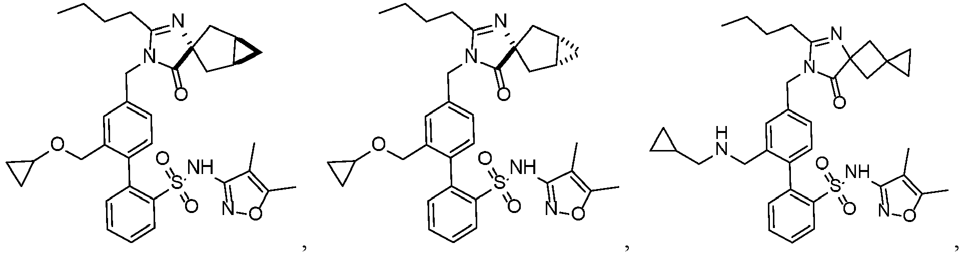

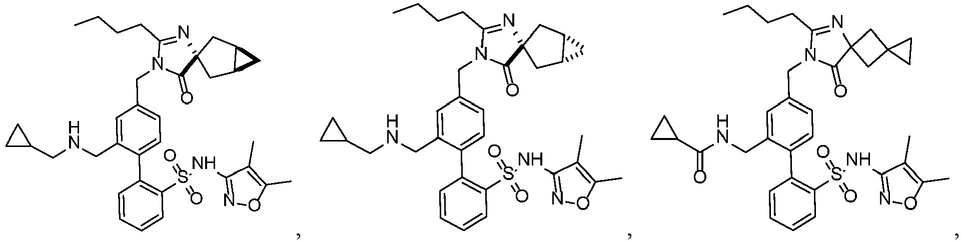

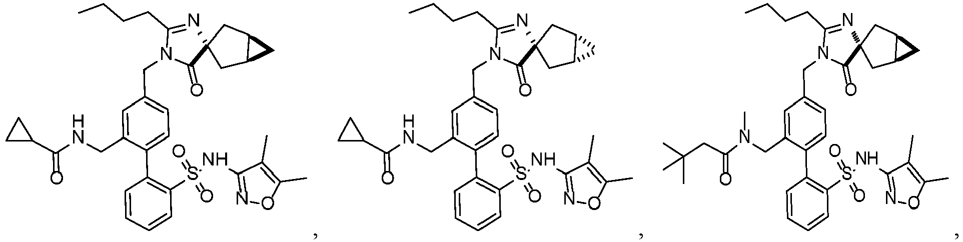

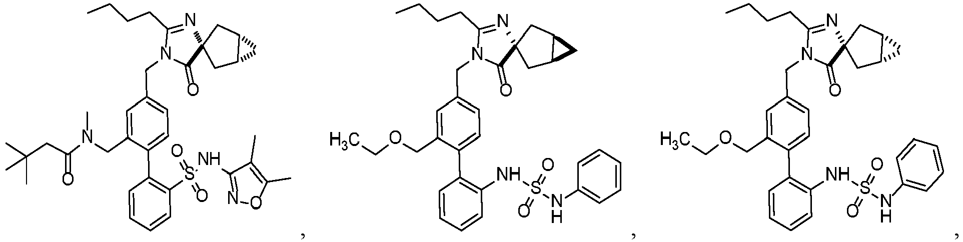

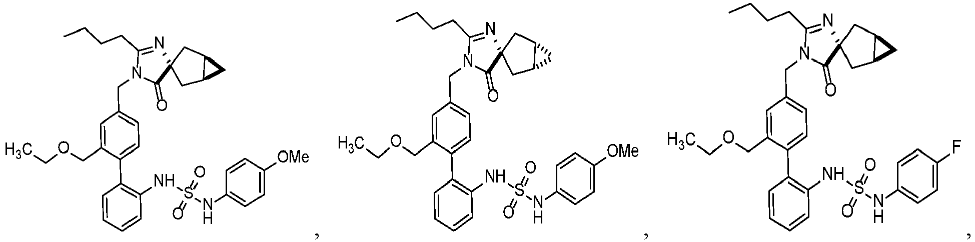

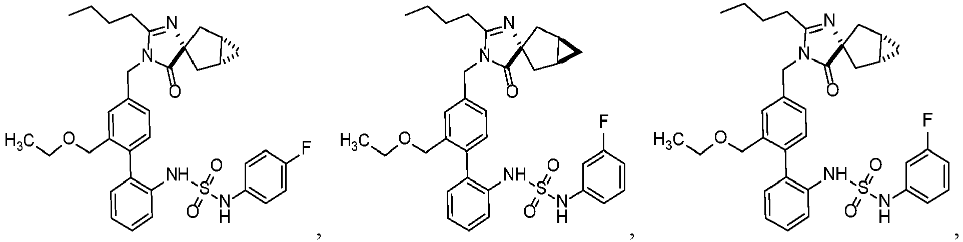

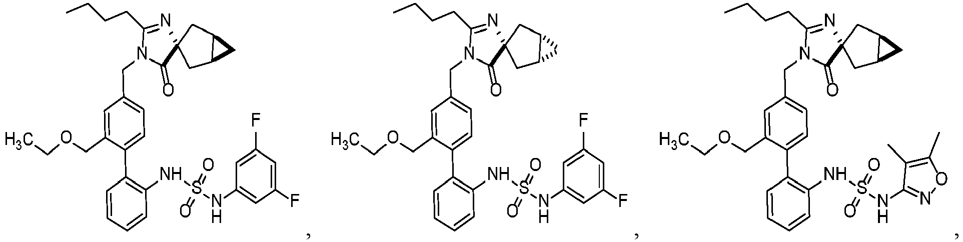

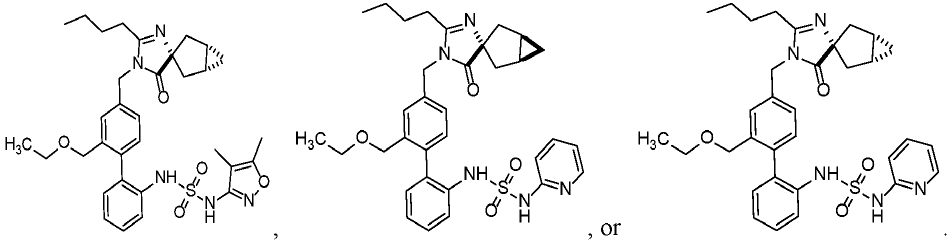

The present disclosure further provides use of any one of the compounds, or the deuterated compounds thereof, or the stereoisomers thereof, or the pharmaceutically acceptable salts thereof as described above in the manufacture of a medicament as dual angiotensin II receptor and endothelin receptor antagonist.

The present disclosure further provides use of any one of the compounds, or the deuterated compounds thereof, or the stereoisomers thereof, or the pharmaceutically acceptable salts thereof as described above in the manufacture of a medicament for treating cardiovascular and cerebrovascular diseases including hypertension, kidney diseases, and diseases associated with organ damage in relation to diabetes. The present disclosure further provides a pharmaceutical composition, comprising a formulation prepared from any one of the compounds, or the deuterated compounds thereof, or the stereoisomers thereof, or the pharmaceutically acceptable salts thereof as described above.

The pharmaceutical composition described above further comprises a pharmaceutically acceptable carrier, excipient, or vehicle.

The present disclosure further provides a method for treating a disease or a condition associated with an angiotensin II receptor and an endothelin receptor, the method comprising administering, to a subject in need thereof, an effective amount of any one of the compounds, or the deuterated compounds thereof, or the stereoisomers thereof, or the pharmaceutically acceptable salts thereof as described above in the present disclosure, or any one of the composition as described above; preferably, the disease includes cardiovascular and cerebrovascular diseases including hypertension, kidney diseases, and diseases associated with organ damage in relation to diabetes. The present disclosure further provides a compound represented by formula IIIa or formula IIIb, or a deuterated compound thereof, or a stereoisomer thereof, or a pharmaceutically acceptable salt thereof: 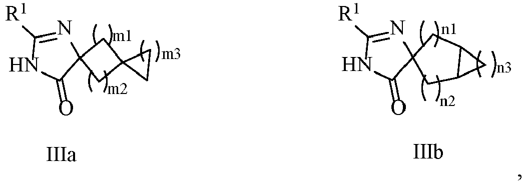 wherein m1, m2, m3, n1, n2, and n3 are as defined above in the present disclosure; R¹ is selected from the group consisting of -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen, preferably n-butyl; m1 and m2 are each independently 0, 1, 2 or 3; and m3 is 1, 2, 3, 4 or 5; n1 and n2 are each independently 0, 1, 2 or 3; and n3 is 1, 2, 3, 4 or 5. Preferably, the compound represented by formula IIIa or formula IIIb is

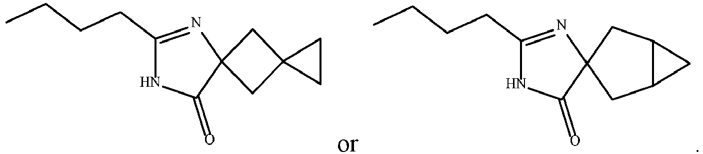

The compounds and derivatives provided in the present disclosure may be named according to the IUPAC (International Union of Pure and Applied Chemistry) or CAS (Chemical Abstracts Service, Columbus, OH) nomenclature system.

Definitions of the terms used in the present disclosure: The initial definition provided for a group or term herein applies to that group or term throughout the specification, unless otherwise stated. Terms without being specifically defined herein shall be given the meanings that a person skilled in the art is able to give based on the disclosure and the context.

"Substituted" refers to a situation in which a hydrogen atom in a molecule is replaced with a different atom or group; or a situation in which lone-pair electrons on a hydrogen atom in a molecule are replaced with an additional atom or group, for example, the lone-pair electrons on S atom may be substituted with O atom(s) to form

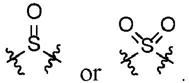

The phrase "optionally further substituted" means that the "substituted" situation may, but does not necessarily, occur and this description involves the case where the substitution occurs and the case where the substitution does not occur.

The minimum and maximum number of carbon atoms in a hydrocarbon group is indicated by a prefix, for example, the prefix C_{a-b} alkyl indicates any alkyl containing the number of carbon atoms from "a" to "b". Thus, for example, C₁₋₆ alkyl refers to alkyl containing the number of carbon atoms from 1 to 6.

"Alkyl" refers to a group originated from a saturated hydrocarbon chain having the specified number of member atoms. Alkyl groups may be linear or branched. Representative branched alkyl groups have one, two or three branches. An alkyl group is optionally substituted with one or more substituents as defined herein. The alkyl group includes methyl, ethyl, propyl (n-propyl and isopropyl), butyl (n-butyl, isobutyl, and tert-butyl), pentyl (n-pentyl, isopentyl, and neopentyl), and hexyl. An alkyl group may also be a moiety of an additional group such as -O(C₁₋₆ alkyl).

"Alkylene" refers to a divalent saturated aliphatic hydrocarbyl having the specified number of member atoms. C_{a-b} alkylene refers to an alkylene group having the number of carbon atoms from a to b. The alkylene group involves branched and linear hydrocarbyl groups. For example, the term "propylidene" may be exemplified by the following structure: 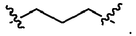 Likewise, the term "dimethylbutylidene" may be, for example, exemplified by either of the following structures:

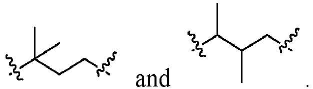

The -C₀₋₄ alkylene in the present disclosure may be C₀ alkylene, C₁ alkylene (*e.g.* -CH₂-), C₂ alkylene (*e.g.* -CH₂CH₂-), C₃ alkylene or C₄ alkylene. C₀ alkylene means that the group here is absent and the two ends thereof are linked via a chemical bond, *e.g.* A-C₀ alkylene-B refers to A-B, namely, the A group is linked directly to the B group via a chemical bond.

"Carbocyclyl" according to the present disclosure refers to a saturated or partially saturated non-aromatic cyclic group having multiple cyclic carbon atoms without cyclic heteroatoms and having a single ring or multiple (fused, bridged, spiro) rings. The term "carbocyclyl" includes cycloalkenyl groups such as cyclohexenyl. Examples of the monocarbocyclyl group include, for instance, cyclopropyl, cyclobutyl, cyclohexyl, cyclopentyl, cyclooctyl, cyclopentenyl, and cyclohexenyl. Examples of the carbocyclyl group with a fused carbocyclyl system include bicyclic hexyl, bicyclic pentyl, and bicyclic octyl. Two such bicyclic alkyls with polycyclic structures are exemplified and named below: 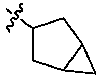 bicyclic hexyl and 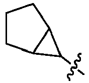 bicyclic hexyl. Examples of the carbocyclyl group with a bridged carbocyclyl system include 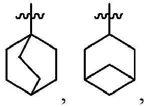 and adamantyl. Examples of the carbocyclyl group with a spiro-carbocyclyl system include 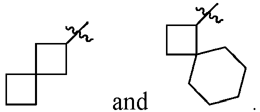 The term "carbocyclyl" further includes a partially saturated cyclic group formed by fusion of an aromatic ring to a non-aromatic ring, where a non-aromatic carbon atom or an aromatic carbon atom may act as a linking position, examples of which include 1,2,3,4-tetrahydronaphthalen-5-yl and 5, 6,7, 8-tetrahydronaphthalen-5-yl.

The unsaturated situation according to the present disclosure means that a group or molecule contains a carbon-carbon double bond, a carbon-carbon triple bond, a carbon-oxygen double bond, a carbon-sulfur double bond, a carbon-nitrogen triple bond, etc.

"Alkenyl" refers to a linear or branched hydrocarbyl group having at least one unsaturated ethylene moiety (>C=C<). For example, C_{a-b} alkenyl refers to an alkenyl group having the number of carbon atoms from a to b and is intended to include, for example, vinyl, propenyl, isopropenyl, and 1,3-butadienyl.

"Alkynyl" refers to a monovalent linear or branched hydrocarbyl containing at least one triple bond. The term "alkynyl" is further intended to include hydrocarbyl groups having one triple bond and one double bond. For example, C₂₋₆ alkynyl is intended to include ethynyl and propynyl.

"Heterocycloalkyl" according to the present disclosure refers to a saturated or partially saturated non-aromatic ring containing at least one heteroatom and having a single ring or multiple (fused, bridged, spiro) rings. The heteroatom refers to a nitrogen atom, an oxygen atom, a sulfur atom, etc. A monovalent saturated or partially unsaturated monocyclic or polycyclic ring system generally indicating multiple ring atoms contains 1, 2 or 3 cyclic heteroatoms selected from N, O and S, while the remaining ring atoms are carbon. Examples of the heterocycloalkyl group in the monoheterocycloalkyl system are oxetanyl, azetidinyl, pyrrolidinyl, 2-oxo-pyrrolidin-3-yl, tetrahydrofuranyl, tetrahydro-thienyl, pyrazolidinyl, imidazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl or oxazepanyl. Examples of the heterocycloalkyl group in the fused heterocycloalkyl system include 8-aza-bicyclo[3.2.1]octyl, quinuclidinyl, 8-oxa-3-aza-bicyclo[3.2.1]octyl, and 9-aza-bicyclo[3.3.1]nonyl. Examples of the heterocycloalkyl group in the bridged heterocycloalkyl system include 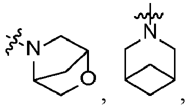 and 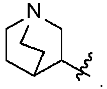 Examples of the heterocycloalkyl group in the spiro-heterocycloalkyl system include 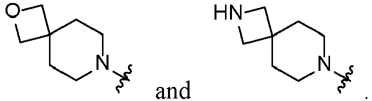 Examples of partially saturated heterocycloalkyl are dihydrofuranyl, imidazolinyl, tetrahydro-pyridyl or dihydropyranyl. The term "heterocycloalkyl" also includes partially saturated cyclic groups containing at least one heteroatom formed by fusion of aromatic rings to non-aromatic rings, where a non-aromatic carbon atom, aromatic carbon atom or heteroatom may act as a linking position, examples of which include

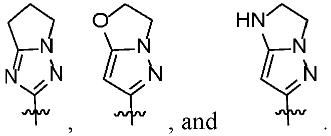

"Aromatic ring" according to the present disclosure refers to an aromatic hydrocarbon group having multiple carbon atoms. Aryl is typically a monocyclic, bicyclic or tricyclic aryl having multiple carbon atoms. Furthermore, the term "aryl" as used herein refers to an aromatic substituent that may be a single aromatic ring or multiple aromatic rings fused together. Non-limiting examples of aryl include phenyl, naphthyl or tetrahydronaphthyl.

"Heteroaromatic ring" according to the present disclosure refers to an aromatic unsaturated ring containing at least one heteroatom. The heteroatom refers to a nitrogen atom, an oxygen atom, a sulfur atom, etc. The heteroaromatic ring may be an aromatic monocyclic or bicyclic hydrocarbon that generally contains multiple ring atoms, one or more of which are heteroatoms selected from O, N, and S, preferably contains one to three heteroatoms. Representatives of heteroaryl include, for example: pyridyl, indolyl, quinoxalinyl, quinolyl, isoquinolinyl, benzothienyl, benzofuranyl, benzothienyl, benzopyranyl, benzothiopyranyl, furyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl, and benzoxazolyl.

"Halogen" according to the present disclosure refers to fluorine, chlorine, bromine or iodine.

"Alkyl substituted with halogen" according to the present disclosure means that one or more hydrogen atoms in alkyl are substituted with halogen. For example, C₁₋₄ alkyl substituted with halogen refers to alkyl containing the number of carbon atoms from 1 to 4, in which hydrogen is substituted with one or more halogen atoms. Another example may include monofluoromethyl, difluoromethyl, and trifluoromethyl.

The "-OR", "-NRR" or the like according to the present disclosure means that the R group is linked to the oxygen atom or the nitrogen atom via a single bond.

The oxygen atom in the "-C(O)R", "-S(O)₂R" or the like according to the present disclosure is linked to the carbon atom or the sulfur atom via a double bond.

The oxygen atom in the "-C(O)R", "-S(O)₂R" or the like according to the present disclosure is linked to the carbon atom or the sulfur atom via a double bond, and the R group is linked to the oxygen atom or the sulfur atom via a single bond. For example, "-S(O)(NH)R" means that the oxygen atom and the nitrogen atom are each linked to the sulfur atom via a double bond, and the R group is linked to the sulfur atom via a single bond. 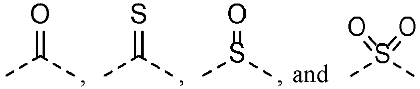 according to the present disclosure means that the oxygen atom or the sulfur atom is linked to the substitution position via a double bond. "---" and " 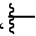 " present in descriptions of the groups in the present disclosure are used to describe the positions where the groups are substituted. For example, 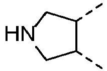 means that the tetrahydropyrrole ring is fused to other ring(s) in the molecular structure via the " position.

The "chemical bond" present in descriptions of the present disclosure refers to a single bond, taking 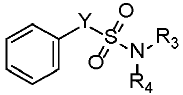 as an example, when Y is a chemical bond, the benzene ring is linked directly to the sulfur atom of the sulfonyl group via a single bond. " 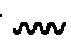 " present in descriptions of the groups in the present disclosure is used to indicate a single stereo-configuration of the chemical structure; for example, " 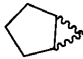 " indicates that the cyclopropane moiety has a single stereo-configuration and the absolute configuration thereof is uncertain.

"Deuterated compound" of the present disclosure means that one or more hydrogen atoms in a molecule or group are substituted with deuterium atom(s), in which the proportion of the deuterium atom(s) is greater than the abundance of deuterium in nature.

The term "pharmaceutically acceptable" means that a carrier, a vehicle, a diluent, an excipient and/or salt(s) formed are generally chemically or physically compatible with the other ingredients of a certain pharmaceutical dosage form and are physiologically compatible with the recipients.

The terms "salt" and "pharmaceutically acceptable salt" refer to acidic and/or basic salts formed by the above-mentioned compounds or stereoisomers thereof with inorganic and/or organic acids and bases, which also include zwitterionic salts (inner salts) and further include quaternary ammonium salts, such as alkylammonium salts. These salts may be obtained directly from the final separation and purification of the compounds. They may also be obtained by appropriately mixing the above compounds or stereoisomers thereof with certain amounts (e.g., equivalent amounts) of acids or bases. These salts may be formed as precipitates in the solutions and collected by filtration, or may be recovered after solvent evaporation, or may be obtained by freeze-drying after reaction in an aqueous medium.

In some embodiments, one or more compounds of the present disclosure may be used in combination. Alternatively, the compounds of the present disclosure may also be used in combination with any other active ingredient to prepare drugs or pharmaceutical compositions for regulating cell functions or treating diseases. If a set of compounds is used, these compounds may be administered to the subject simultaneously, separately or sequentially.

Apparently, based on the above contents of the present disclosure, various other modifications, substitutions or alternations may also be made according to general technical knowledge and customary means in the art, without departing from the above basic technical ideas of the present disclosure.

The above contents of the present disclosure will be further described in detail below with reference to the specific embodiments by means of examples, which, however, shall not be construed as the scope of the above subject matters of the present disclosure being limited only to the following examples. Any technologies implemented on the basis of the contents described above fall within the scope of the present disclosure.

### BRIEF DESCRIPTION OF THE DRAWINGS

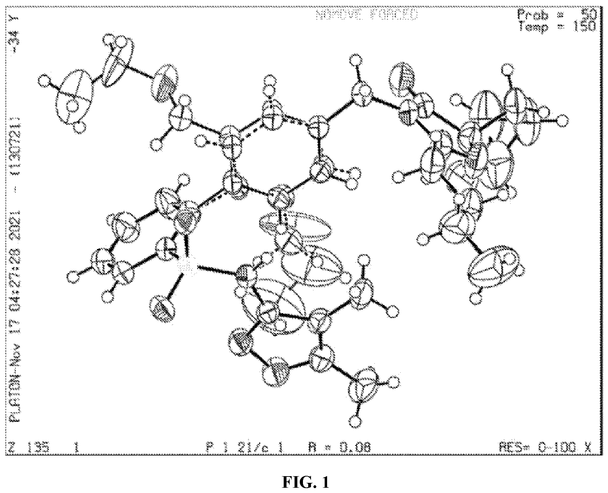

FIG. 1 shows a schematic diagram for the three-dimensional structure of the compound in Example 3 of the present disclosure obtained by the analysis method of single crystal X-ray diffraction.

### DETAILED DESCRIPTION

The structures of compounds are determined by nuclear magnetic resonance (NMR) and mass spectrometry (MS). For NMR, shift (δ) is given in the unit of 10⁻⁶ (ppm). The NMR measurement is performed by using nuclear magnetic instruments (Bruker AvanceIII 400 and Bruker Avanceneo 600). The solvents for measurement are deuterated dimethyl sulfoxide (DMSO-*d₆*), deuterated chloroform (CDCl₃), and deuterated methanol (CD₃OD). The internal standard is tetramethylsilane (TMS).

Shimadzu's liquid chromatograph mass spectrometer (Shimadzu LC-MS 2020 (ESI)) is used for the LC-MS measurement. Shimadzu's high pressure liquid chromatograph (Shimadzu LC-20A) is used for the HPLC measurement. Gilson GX-281 reversed-phase preparative chromatograph is used for MPLC (medium pressure preparative chromatography). Silica gel plates for thin layer chromatography are Yantai Huanghai HSGF254 or Qingdao GF254 silica gel plates, and the specifications of products for separation and purification by the thin layer chromatography vary from 0.4 mm to 0.5 mm. Generally, 200 to 300-mesh silica gel of Yantai Huanghai Silica Gel is used as support in column chromatography.

Known starting materials in the present disclosure may be synthesized by or according to the methods known in the art, or may be purchased from supply corporations, e.g. Energy Chemical, Chengdu Kelong Chemical, Accela ChemBio, and J&K Scientific.

The reactions are carried out under a nitrogen atmosphere unless otherwise indicated in the Examples. The solutions refer to aqueous solutions unless otherwise indicated in the Examples. The reaction temperature is room temperature unless otherwise indicated in the Examples. Mole per liter is abbreviated to M unless otherwise indicated in the Examples.

DIBAL-H: diisobutyl aluminium hydride; DPPA: diphenylphosphoryl azide; DMF: dimethylformamide.

### Example 1

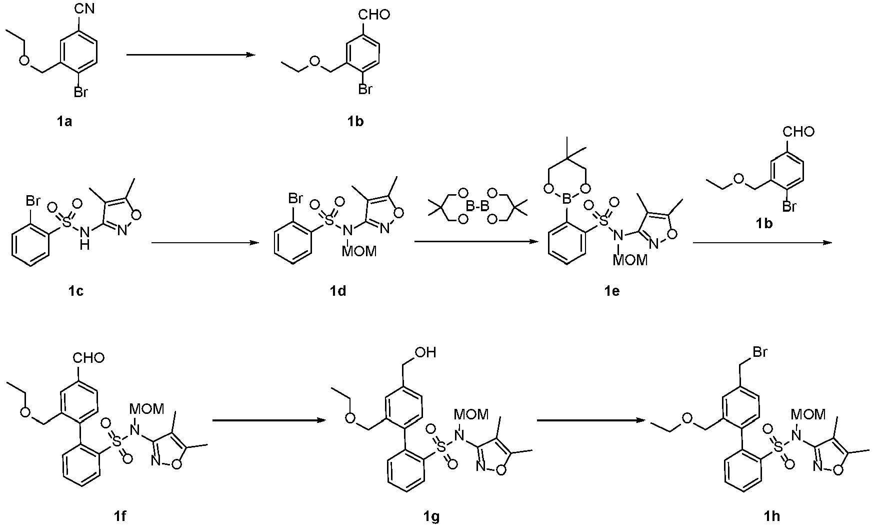

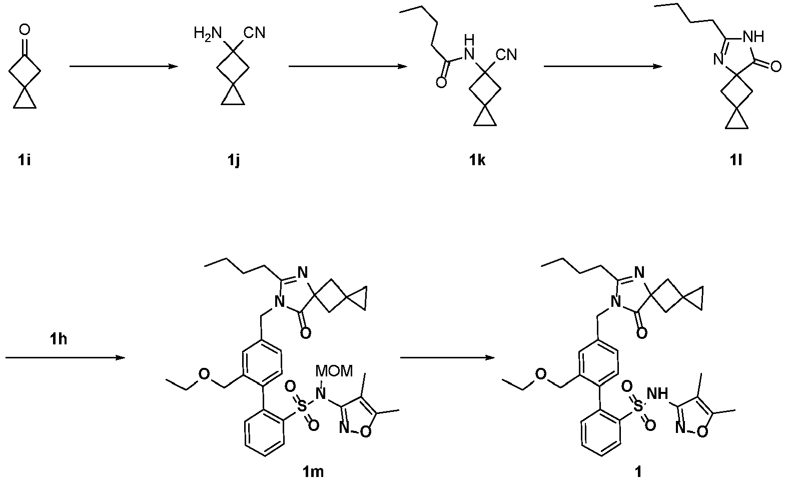

### Step 1

Compound **1a** (300 mg, 1.25 mmol) was dissolved in anhydrous tetrahydrofuran (5 mL). After the system was cooled to -60°C, a solution of DIBAL-H in n-hexane (1 **M**, 1.62 mL, 1.62 mmol) was added dropwise. The reaction system was heated to room temperature and reacted for 2 hours. After the completion of the reaction was monitored by LC-MS, the reaction mixture was poured into dilute hydrochloric acid (2 **M**, 10 mL). The resultant was extracted with ethyl acetate (20 mL*3). The organic phases were combined, washed with saturated saline (10 mL), dried over anhydrous sodium sulfate, and then concentrated under reduced pressure. The resulting crude product was purified by column chromatography (petroleum ether:ethyl acetate=9:1) to afford compound **1b** (200 mg, 0.82 mmol, yield: 66%).

### Step 2

Compound **1c** (3.50 g, 10.57 mmol) was dissolved in 50 mL of anhydrous tetrahydrofuran. Sodium hydride (60%, 592 mg, 14.80 mmol) was added at 0°C and stirred for 30 min at this temperature. Thereafter, bromomethyl methyl ether (1.85 g, 14.80 mmol) was added. Afterwards, the reaction system was heated to room temperature and stirred for 16 hours. The reaction system was poured into 50 mL of saturated aqueous ammonium chloride solution, and extracted with ethyl acetate (30 mL*3). The organic phases were combined, washed with 50 mL of saturated saline, dried over anhydrous sodium sulfate, concentrated to dryness under reduced pressure, and purified by column chromatography (petroleum ether:ethyl acetate=4:1) to afford compound **1d** (3.20 g, 8.53 mmol, yield: 81%). MS-ESI calc. for 397.0 [M+Na]⁺, found 397.0. ¹H NMR (400 MHz, DMSO-*d₆*) *δ* 7.98 - 7.96 (m, 1H), 7.92 - 7.89 (m, 1H), 7.61 - 7.54 (m, 2H), 5.16 (s, 2H), 3.39 (s, 3H), 2.32 (s, 3H), 1.85 (s, 3H).

### Step 3

Compound **1d** (300 mg, 0.80 mmol) was dissolved in 1,4-dioxane (9 mL). After 5 min following the bubbling of nitrogen gas into the reaction system, bis(neopentyl glycolato)diboron (304 mg, 1.35 mmol), Pd(dppf)Cl₂ (29.0 mg, 0.040 mmol), and potassium acetate (157 mg, 1.60 mmol) were added. The reaction system was heated and refluxed for 3 hours. After the completion of the reaction was monitored by LC-MS, the system was filtered, concentrated under reduced pressure, and purified by column chromatography (petroleum ether:ethyl acetate=4:1) to afford compound **1e** (250 mg, 0.61 mmol, yield: 77%). ¹H NMR (400 MHz, DMSO-*d₆*) *δ* 7.70 - 7.52 (m, 4H), 4.95 (s, 2H), 3.66 (s, 4H), 3.27 (s, 3H), 3.27 (s, 3H), 2.34 (s, 3H), 1.81 (s, 3H). 1.03 (s, 6H).

### Step 4

Under the protection of nitrogen gas, compound **1e** (500 mg, 1.18 mmol) was dissolved in a mixed solution of toluene (2 mL) and ethanol (1 mL), and then tetrakis(triphenylphosphine)palladium (137 mg, 0.118 mmol) and sodium carbonate (377 mg, 3.56 mmol) were added. The reaction system was heated to 85°C and reacted for 3 hours. After cooling to room temperature, the reaction system was extracted with ethyl acetate (20 mL*3). The organic phases were combined, washed with saturated saline (10 mL), dried over anhydrous sodium sulfate, then concentrated under reduced pressure, and purified by column chromatography (petroleum ether:ethyl acetate=7:3) to afford compound **1f** (225 mg, 0.49 mmol, yield: 42%). MS-ESI calc. for 481.1 [M+Na]⁺, found 481.2. ¹H NMR (400 MHz, DMSO-*d*₆) *δ* 10.09 (s, 1H), 8.04 - 7.96 (m, 2H), 7.84 (dd, *J* = 7.8, 1.7 Hz, 1H), 7.76 (td, *J* = 7.5, 1.4 Hz, 1H), 7.66 (td, *J* = 7.8, 1.4 Hz, 1H), 7.37 - 7.29 (m, 2H), 4.53 (d, *J* = 11.0 Hz, 1H), 4.39 (d, *J* = 11.0 Hz, 1H), 4.24 (d, *J* = 12.9 Hz, 1H), 4.13 (d, *J* = 12.8 Hz, 1H), 3.42 - 3.23 (m, 2H), 3.20 (s, 3H), 2.33 (s, 3H), 1.79 (s, 3H), 1.05 (t, *J* = 7.0 Hz, 3H).

### Step 5

After compound **1f** (200 mg, 0.436 mmol) was dissolved in methanol (2 mL), the reaction system was cooled to 0°C, and sodium borohydride (60%, 24.8 mg, 0.62 mmol) was added. Thereafter, the reaction system was heated to room temperature and reacted for 2 hours. After the completion of the reaction was detected by LC-MS, the reaction system was poured into a dilute hydrochloric acid solution (0.5 **M**, 10 mL), and extracted with ethyl acetate (20 mL*3). The organic phases were combined and washed with saturated saline (10 mL), dried over anhydrous sodium sulfate, then concentrated under reduced pressure, and subjected to column chromatography (petroleum ether:ethyl acetate=4:1) to afford 150 mg of compound **1g** (150 mg, 0.326 mmol, yield: 75%). MS-ESI calc. for 483.2 [M+Na]⁺, found 483.2. ¹H NMR (400 MHz, Chloroform-*d*) *δ* 8.00 (dd, *J* = 8.1, 1.3 Hz, 1H), 7.62 - 7.53 (m, 2H), 7.45 (td, *J* = 7.6, 1.4 Hz, 1H), 7.37 - 7.27 (m, 3H), 4.76 (s, 2H), 4.37 (dd, *J* = 11.6, 5.9 Hz, 2H), 4.19 (dd, *J* = 18.0, 11.6 Hz, 2H), 3.50 - 3.27 (m, 2H), 3.32 (s, 3H), 2.30 (s, 3H), 1.92 (s, 3H), 1.12 (t, *J* = 7.0 Hz, 3H).

### Step 6

At room temperature, compound **1g** (150 mg, 0.326 mmol) was dissolved in N,N-dimethylformamide (0.5 mL). The reaction system was cooled to 0°C, and tetrabromomethane (162 mg, 0.488 mmol) and triphenylphosphine (128 mg, 0.488 mmol) were added, and reacted at 0°C for 2 hours. After the completion of the reaction was detected by LC-MS, the reaction mixture was concentrated under reduced pressure, and purified by column chromatography (petroleum ether:ethyl acetate=4:1) to afford compound **1h** (122 mg, 0.233 mmol, yield: 71%). MS-ESI calc. for 547.1 [M+Na]⁺, found 547.1. ¹H NMR (400 MHz, Chloroform-*d*) *δ* 8.00 (dd, *J* = 8.0, 1.3 Hz, 1H), 7.62 - 7.55 (m, 5H), 7.49 - 7.41 (m, 2H), 7.33 - 7.25 (m, 7H), 4.56 (s, 2H), 4.41 - 4.25 (m, 2H), 4.22 - 4.12 (m, 2H), 3.50 - 3.28 (m, 2H), 3.31 (s, 3H), 2.30 (s, 3H), 1.92 (s, 3H), 1.13 (t, *J* = 7.0 Hz, 3H).

### Step 7

Compound **1i** (200 mg, 2.08 mmol) was dissolved in isopropanol (0.2 mL). Afterwards, the above solution was carefully added dropwise into a hydroxylamine solution (0.3 mL) containing ammonium chloride (145 mg, 2.71 mmol) and potassium cyanide (169 mg, 2.60 mmol). The reaction system was reacted at room temperature for 16 hours. After the completion of the reaction was detected by TLC, the reaction system was poured into water (10 mL). The aqueous phase was extracted with dichloromethane (10 mL*3). The organic phases were combined, washed with saturated saline, dried over anhydrous sodium sulfate, and concentrated to afford 205 mg of compound **1j** (205 mg, 1.68 mmol, yield: 81%). ¹H NMR (400 MHz, Chloroform-*d*) *δ* 3.00 - 2.27 (m, 6H), 0.63 - 0.46 (m, 4H).

### Step 8

Compound **1j** (600 mg, 4.91 mmol) was dissolved in toluene (6 mL), and triethylamine (745 mg, 7.36 mmol) was added. At 15°C, n-pentanoyl chloride (711 mg, 5.90 mmol) was added slowly, and the reaction system was reacted at 80°C for 2 hours. After the completion of the reaction was detected by TLC, the reaction system was poured into water (8 mL), and a dilute hydrochloric acid solution (0.2 **M**, 8 mL) was added. The organic phase was separated and washed with water (10 mL). The organic phase was concentrated to afford compound **1k** (820 mg of crude product), which was directly used in the next step.

### Step 9

At 20°C, compound **1k** (820 mg, the crude product obtained in the previous step) was dissolved in methanol (5 mL), and potassium hydroxide (172 mg, 3.07 mmol) and hydrogen peroxide (30%, 2 mL, 4.91 mmol) were added. After reacting at 50°C for 30 min, the reaction system was cooled to room temperature and potassium hydroxide (689 mg, 12.28 mmol) was added again. The reaction system was heated to 70°C and reacted for 2 hours. After the completion of the reaction was detected by LC-MS or TLC, solid ammonium chloride (100 mg) was added to the reaction system for neutralization. The reaction system was concentrated under reduced pressure and extracted with ethyl acetate (10 mL). The organic phase was washed with saturated saline (10 mL), dried over anhydrous sodium sulfate, concentrated under reduced pressure, and purified by column chromatography (petroleum ether:ethyl acetate=7:3) to afford compound **1l** (203 mg, 0.984 mmol, two-step yield: 20%). MS-ESI calc. for 207.3 [M+H]⁺, found 207.2. ¹H NMR (400 MHz, Chloroform-*d*) *δ* 2.64 - 2.56 (m, 2H), 2.55 - 2.41 (m, 4H), 1.68 (p, *J* = 7.5 Hz, 2H), 1.51 - 1.34 (m, 3H), 0.95 (t, *J* = 7.3 Hz, 3H), 0.66 - 0.51 (m, 4H).

### Step 10

Compound **1l** (50 mg, 0.242 mmol) was dissolved in N,N-dimethylformamide (2 mL). The system was cooled to 0°C, and sodium hydride (60%, 12.2 mg, 0.51 mmol) was added carefully and stirred for 30 min. Subsequently, the solution of the intermediate **1h** (114 mg, 0.218 mmol) in N,N-dimethylformamide (0.5 mL) was added to the system. The system was returned to room temperature and reacted for 5 hours. After the completion of the reaction was monitored by LC-MS, the system was poured into a saturated ammonium chloride (10 mL), and extracted with ethyl acetate (20 mL). The organic phase was washed with saturated saline (10 mL), dried over anhydrous sodium sulfate, concentrated under reduced pressure, and purified by column chromatography (petroleum ether:ethyl acetate=4:1) to afford 80 mg of compound **1m** (80 mg, 0.123 mmol, yield: 51%). MS-ESI calc. For 649.8 [M+H]⁺, found 649.4. ¹H NMR (400 MHz, DMSO-*d₆*) *δ* 7.93 (dd, *J* = 8.1, 1.3 Hz, 1H), 7.73 - 7.67 (m, 1H), 7.61 - 7.55 (m, 1H), 7.31 - 7.25 (m, 2H), 7.14 - 7.08 (m, 2H), 4.75 (s, 2H), 4.29 (d, *J* = 11 Hz, 1H), 4.19 - 4.13 (m, 2H), 4.05 (d, *J* = 12.9 Hz, 1H), 3.31 - 3.15 (m, 2H), 3.17 (s, 3H), 2.48 - 2.34 (m, 6H), 2.31 (s, 3H), 1.79 (s, 3H), 1.59 - 1.51 (m, 2H), 1.35 - 1.27 (m, 2H), 1.0 (t, *J* = 7 Hz, 3H), 0.84 (t, *J* = 4.4 Hz, 3H), 0.63 - 0.49 (m, 4H).

### Step 11

Compound **1m** (70 mg, 0.108 mmol) was dissolved in a mixed solution of ethanol (3 mL) and hydrochloric acid (6 **M**, 3 mL), and reacted at 75°C for 3 hours. After the completion of the reaction, the pH value of the system was adjusted to 8 with a sodium hydroxide solution (5 **M**) and then adjusted to 5 with dilute hydrochloric acid (0.5 **M**). The system was extracted with ethyl acetate (20 mL*3), and the organic phases were combined, washed with saturated saline (20 mL), dried over anhydrous sodium sulfate, then concentrated under reduced pressure, and purified by column chromatography (petroleum ether:ethyl acetate=4:1) to afford compound **1** (37 mg, 0.061 mmol, yield: 56%, HPLC purity: 98.6%). MS-ESI calc. for 605.8 [M+H]⁺, found 605.4. ¹H NMR (400 MHz, DMSO-*d₆*) *δ* 10.48 (s, 1H), 8.08 - 8.03 (m, 1H), 7.68 - 7.58 (m, 2H), 7.23 - 7.15 (m, 2H), 7.02 (dd, *J* = 1.6, 1.6 Hz, 1H), 6.93 (d, *J* = 7.8 Hz, 1H), 4.72 (s, 2H), 4.00 (s, 2H), 3.25 - 3.14 (m, 2H), 2.49 - 2.46 (m, 2H), 2.42 - 2.35 (m, 4H), 2.2 (s, 3H), 1.66 (s, 3H), 1.6 - 1.5 (m, 2H), 1.35 - 1.26 (m, 2H), 0.99 (t, *J* = 6.9 Hz, 3H), 0.83 (t, *J* = 7.2 Hz, 3H), 0.62 - 0.49 (m, 4H).

### Example 2

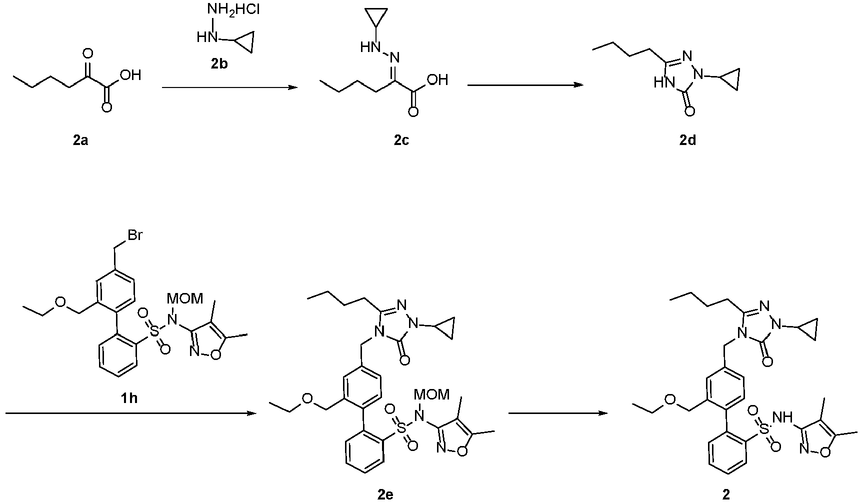

### Step 1

At 25°C, compound **2b** (350 mg, 3.22 mmol) was dissolved in 1 ml of water, and a hydrochloric acid solution (0.5 N, 0.35 mL) was added dropwise into the system. After continuously stirring for 5 min, an aqueous solution (1 mL) of compound **2a** (460 mg, 3.53 mmol) was added dropwise into the system. After stirring for 4 hours, ethanol was added to the system until the system was clear, and the reaction solution was frozen at 4°C for 16 hours. After the system was fed to LC-MS for monitoring the completion of the reaction, the system was extracted with ethyl acetate (15 mL*3). The organic phases were combined and washed with saturated saline (50 mL), dried over anhydrous sodium sulfate, and concentrated under reduced pressure to afford compound **2c** (500 mg), which was used directly in the next step.

MS-ESI calc. for 185.1 [M+H]⁺, found 185.2.

### Step 2

At room temperature, triethylamine (1.5 mL, 10.8 mmol) and DPPA (710 mg, 2.58 mmol) were added to a solution of compound **2c** (500 mg, the crude product obtained in the previous step) in toluene (6 mL), and the system was refluxed at 120°C for 1 hour. After the completion of the reaction was monitored by LC-MS, the system was poured into water (8 mL), and extracted with ethyl acetate (10 mL*3). The organic phases were combined, washed with water, dried over anhydrous sodium sulfate, concentrated under reduced pressure, and purified by column chromatography (water/acetonitrile) to afford compound **2d** (398 mg, 2.20 mmol, total two-step yield: 68%). MS-ESI calc. for 182.1 [M+H]⁺, found 182.1. ¹H NMR (600 MHz, Chloroform-*d*) *δ* 11.67 (s, 1H), 3.12 (tt, *J* = 7.1, 3.7 Hz, 1H), 2.51 (t, *J* = 7.8 Hz, 2H), 1.65 (p, *J* = 7.7 Hz, 2H), 1.38 (h, *J* = 7.4 Hz, 2H), 1.04 (q, *J* = 3.9 Hz, 2H), 1.02 - 0.91 (m, 5H).

### Step 3

In an ice bath, sodium hydride (60%, 12 mg, 0.50 mmol) was added to a solution of compound **2d** (30 mg, 0.166 mmol) in DMF (2 mL). After the system was reacted at 25°C for 30 min, a solution of compound **1h** (80 mg, 0.153 mmol) in DMF (0.5 mL) was added dropwise into the reaction system, and further stirred for 5 hours. After the completion of the reaction was monitored by LC-MS, the system was poured into saturated ammonium chloride (10 mL). The aqueous phase was extracted with ethyl acetate (10 mL). The organic phase was separated and washed with saturated saline (10 mL), dried over anhydrous sodium sulfate, concentrated under reduced pressure, and purified by column chromatography (petroleum ether:ethyl acetate=4:1) to afford compound **2e** (80 mg, 0.128 mmol, yield: 84%). ¹H NMR (600 MHz, DMSO-*d*₆) *δ* 7.93 (dd, *J* = 8.1, 1.2 Hz, 1H), 7.71 (td, *J* = 7.6, 1.3 Hz, 1H), 7.59 (td, *J* = 7.8, 1.4 Hz, 1H), 7.33 (d, *J* = 1.7 Hz, 1H), 7.28 (dd, *J* = 7.6, 1.4 Hz, 1H), 7.17 - 7.09 (m, 2H), 4.87 (s, 2H), 4.31 (d, *J* = 11.0 Hz, 1H), 4.20 - 4.14 (m, 2H), 4.06 (d, *J* = 12.9 Hz, 1H), 3.34 - 3.11 (m, 3H), 3.17 (s, 3H), 2.44 - 2.38 (m, 2H), 2.31 (s, 3H), 1.79 (s, 3H), 1.51 - 1.42 (m, 2H), 1.33 - 1.21 (m, 2H), 1.01 (t, *J* = 7.0 Hz, 3H), 0.94 - 0.85 (m, 4H), 0.83 (t, *J* = 6.0 Hz, 3H).

### Step 4

65 mg of compound **2e** (65 mg, 0.104 mmol) was dissolved in ethanol (3 mL), and a hydrochloric acid solution (6 **M**, 3 mL) was added. The reaction system was reacted at 75°C for 5 hours. When about 85% target product was detected by LC-MS, the pH value of the system was adjusted to 8 with a sodium hydroxide solution (5 **M**), and then adjusted to 5 with a hydrochloric acid solution (0.5 **M**). The aqueous phase was extracted with ethyl acetate (20 mL), and the organic phase was washed with saturated saline, then dried over anhydrous sodium sulfate, concentrated under reduced pressure, and purified by column chromatography (petroleum ether:ethyl acetate=7:3) to obtain the final compound 2 (37 mg, 0.064 mmol, yield: 62%, HPLC purity: 97.4%). MS-ESI calc. for 580.3 [M+H]⁺, found 580.2. ¹H NMR (600 MHz, DMSO-*d*₆) *δ* 10.50 (s, 1H), 8.05 (dd, *J* = 8.0, 1.5 Hz, 1H), 7.68 - 7.58 (m, 2H), 7.27 (d, *J* = 1.8 Hz, 1H), 7.19 (dd, *J* = 7.4, 1.6 Hz, 1H), 7.07 (dd, *J* = 7.8, 1.9 Hz, 1H), 6.94 (d, *J* = 7.8 Hz, 1H), 4.84 (s, 2H), 4.01 (q, *J* = 12.9 Hz, 2H), 3.28 - 3.12 (m, 3H), 2.43 (t, *J* = 7.7 Hz, 2H), 2.20 (s, 3H), 1.67 (s, 3H), 1.47 (q, *J* = 7.7 Hz, 2H), 1.37 - 1.21 (m, 2H), 1.00 (t, *J* = 7.0 Hz, 3H), 0.89 (d, *J* = 5.4 Hz, 4H), 0.83 (t, *J* = 7.3 Hz, 3H).

### Example 3

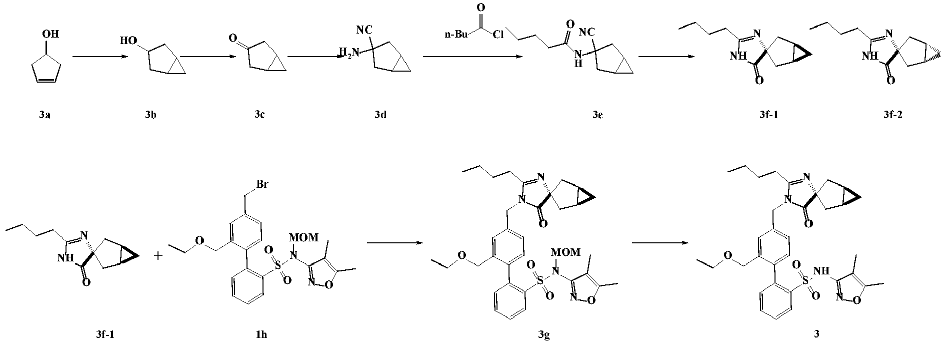

### Step 1

At 0°C, diiodomethane (24.5 g, 91.5 mmol) was carefully added dropwise into a solution of diethyl zinc in dichloromethane (166 mL, 1 **M**, 166 mmol) and stirred for 30 min, and compound **3a** (7.0 g, 83.3 mmol) was added and continued to react for 3 hours. After the completion of the reaction was monitored by TLC, a saturated aqueous ammonium chloride solution (100 mL) was added to quench the reaction. The system was extracted with dichloromethane (200 mL*2). The organic phases were combined, and washed with saturated saline, dried over anhydrous sodium sulfate, concentrated under reduced pressure, and purified by column chromatography (petroleum ether:ethyl acetate=4:1) to afford compound **3b** (2.0 g, 20.4 mmol, yield: 24%). ¹H NMR (400 MHz, Chloroform-*d*) *δ* 4.38 (tt, *J* = 6.6, 0.9 Hz, 1H), 2.16 - 2.04 (m, 2H), 1.72 (d, *J*=12 Hz, 2H), 1.42 (s, 1H), 1.34 - 1.22 (m, 2H), 0.58 - 0.43 (m, 2H).

### Step 2

Compound **3b** (200 mg, 2.0 mmol) was dissolved in dichloromethane (12 mL). The system was cooled to 0°C, and a Dess-Martin oxidant (951 mg, 2.2 mmol) was added. The reaction system was heated to room temperature and reacted for 3 hours. After the completion of the reaction was monitored by TLC, the reaction system was filtered. The filtrate was concentrated and purified by column chromatography (petroleum ether:ethyl acetate=4:1) to afford compound **3c** (70 mg, 0.73 mmol, yield: 36%). ¹H NMR (400 MHz, Chloroform-*d*) *δ* 2.66 - 2.50 (m, 2H), 2.22 - 2.10 (d, *J*=20 Hz, 2H), 1.54 (dtd, *J* = 8.0, 4.0, 1.6 Hz, 2H), 0.91 (tdt, *J* = 7.8, 5.8, 1.9 Hz, 1H), 0.01- -0.03 (m, 1H).

### Step 3

Compound **3c** (890 mg, 9.26 mmol) was dissolved in isopropanol (2 mL), and the mixture was carefully added dropwise into an aqueous ammonia solution (3 mL) containing ammonium chloride (644 mg, 12.04 mmol) and potassium cyanide (754 mg, 11.58 mmol). The reaction system was reacted at room temperature for 16 hours. After the completion of the reaction was detected by TLC, the reaction system was poured into water (15 mL). The aqueous phase was extracted with dichloromethane (20 mL*2). The organic phases were combined, washed with saturated saline, dried over sodium sulfate, and concentrated to afford compound **3d** containing two isomers (800 mg, 6.55 mmol, yield: 71%).

### Step 4

Compound **3d** (400 mg, 3.27 mmol) was dissolved in toluene (5 mL), and triethylamine (497 mg, 4.91 mmol) was added. At 15°C, n-pentanoyl chloride (474 mg, 3.93 mmol) was added carefully, and the reaction system was reacted at 80°C for 2 hours. After the completion of the reaction was detected by TLC, the reaction system was poured into water (8 mL), and a dilute hydrochloric acid solution (0.2 **M**, 8 mL) was added. The organic phase was separated and washed with water (10 mL). The organic phase was separated and concentrated under reduced pressure to obtain a crude product of compound **3e** (610 mg), which was used directly in the next reaction step.

### Step 5

At 20°C, the crude product of compound **3e** (610 mg) was dissolved in methanol (6 mL), and potassium hydroxide (332 mg, 5.92 mmol) and 30% hydrogen peroxide (1.5 mL) were added. The reaction system was reacted at 50°C for 30 min, cooled at room temperature, and added with potassium hydroxide (332 mg, 5.92 mmol) again. The reaction system was heated to 70°C and reacted for 2 hours. After the completion of the reaction was detected by LC-MS or TLC, solid ammonium chloride (1.2 g) was added to the reaction system and stirred, and the reaction system was diluted with methanol and then concentrated. Water (20 mL) was added to the concentrate. The aqueous phase was extracted with ethyl acetate (30 mL). After separation, the organic phase was washed with saturated saline, dried over anhydrous sodium sulfate, concentrated under reduced pressure, purified by column chromatography (petroleum ether:ethyl acetate=7:3), and separated to obtain two isomers **3f-1, 3f-2** (at a ratio of about 1:1) (totally 504 mg, 2.44 mmol, total two-step yield: 75%). The relative configurations of the two resulting isomers were confirmed by the single crystal diffraction experiment in Example 3. The isomer 3f-1was used for Examples 3, 5, and 7.

**3f-1** Rf= 0.3 (ethyl acetate:petroleum ether=7:3). ¹H NMR (400 MHz, Chloroform-*d*) *δ* 2.48 - 2.36 (t, *J*=8 Hz, 2H), 2.25 (ddt, *J* = 14.0, 4.1, 1.2 Hz, 2H), 1.92 (d, *J* = 13.8 Hz, 2H), 1.71 - 1.58 (m, 2H), 1.55 - 1.45 (m, 2H), 1.43 - 1.32 (m, 2H), 0.98 - 0.85 (t, *J*= 8 Hz, 3H), 0.96 - 0.85 (m, 1H), 0.69 - 0.59 (m, 1H).

**3f-2** Rf=0.5 (ethyl acetate:petroleum ether=7:3). ¹H NMR (400 MHz, Chloroform-*d*) *δ* 2.45 - 2.35 (t, *J*=8 Hz, 2H), 2.29 (ddd, *J* = 13.1, 3.1, 1.5 Hz, 2H), 1.79 (d, *J*=12 Hz, 2H), 1.67 - 1.56 (m, 2H), 1.52 - 1.43 (m, 2H), 1.37 (dq, *J* = 14.6, 7.4 Hz, 2H), 0.99 (q, *J* = 4.3 Hz, 14H), 0.92 (t, *J* = 7.3 Hz, 3H), 0.47 (tdt, *J* = 8.2, 4.8, 1.1 Hz, 1H).

### Step 6

Compound **3f-1**(50 mg, 0.24 mmol) was dissolved in N,N-dimethylformamide (2 mL). After cooling to 0°C, sodium hydride (60%, 12 mg, 0.31 mmol) was added and stirred for 30 min, and then a solution of compound **1h** (114 mg, 0.22 mmol) in N,N-dimethylformamide (0.5 mL) was added dropwise and continued to react for 5 hours. After the completion of the reaction was monitored by LC-MS, the system was poured into saturated ammonium chloride (10 mL), and extracted with ethyl acetate (10 mL*3). The organic phases were combined, washed with a saturated sodium chloride solution, dried over anhydrous sodium sulfate, and purified by column chromatography (petroleum ether:ethyl acetate=4:1) to afford compound **3g** (110 mg, 0.17 mmol, yield: 71%). ¹H NMR (400 MHz, DMSO-*d₆*) *δ* 7.93 (dd, *J* = 8.0, 1.3 Hz, 1H), 7.71 (td, *J* = 7.5, 1.4 Hz, 1H), 7.59 (td, *J* = 7.7, 1.4 Hz, 1H), 7.30 (dd, *J* = 7.6, 1.4 Hz, 1H), 7.25 (d, *J* = 1.7 Hz, 1H), 7.17 - 7.07 (m, 2H), 4.71 (s, 2H), 4.28 (d, *J* = 11.1 Hz, 1H), 4.22 - 4.11 (m, 2H), 4.07 (d, *J* = 13.0 Hz, 1H), 3.31 - 3.21 (m, 2H), 3.18 (s, 3H), 2.37 - 2.26 (m, 5H), 2.18 (d, *J* = 3.7 Hz, 2H), 1.85 (dd, *J* = 13.6, 1.5 Hz, 2H), 1.81 (d, *J* = 0.8 Hz, 3H), 1.57 - 1.44 (m, 4H), 1.36 - 1.26 (m, 2H), 1.05 - 0.96 (m, 1H), 1.02-1.05 (t, *J*=7.3 Hz, 3H)0.83 (t, *J* = 7.3 Hz, 3H), 0.55 (td, *J* = 8.2, 4.3 Hz, 1H).

### Step 7

Compound **3g** (50 mg, 0.08 mmol) was dissolved in a mixed solution of 95% ethanol (3 mL) and 6 **M** hydrochloric acid (3 mL), and reacted at 75°C for 3 hours. LC-MS might detect production of 80% of the product. After the completion of the reaction, the pH value of the system was adjusted to 8 with 5 **M** sodium hydroxide solution, and then adjusted to 5 with 0.5 **M** dilute hydrochloric acid. The aqueous phase was extracted with ethyl acetate (20 mL), washed with saturated saline, dried over anhydrous sodium sulfate, and concentrated under reduced pressure. The crude product was purified by reversed-phase medium-pressure preparative chromatography (water-acetonitrile system) and then purified by column chromatography (petroleum ether:ethyl acetate=1:1) to obtain the final compound **3** (30 mg, 0.05 mmol, yield: 63%, HPLC purity: 97.3%). MS-ESI calc. for 605.3 [M+H]⁺, found 605.2. ¹H NMR (400 MHz, DMSO-*d₆*) *δ* 10.48 (s, 1H), 8.09 - 8.03 (m, 1H), 7.63 (pd, *J* = 7.4, 1.6 Hz, 2H), 7.24 - 7.13 (m, 2H), 7.05 - 6.87 (m, 2H), 4.68 (s, 2H), 4.00 (s, 2H), 3.28 - 3.13 (m, 2H), 2.32 (t, *J* = 7.4 Hz, 2H), 2.23 - 2.16 (m, 2H), 2.21 (s, 3H), 1.86 (dd, *J* = 13.6, 1.5 Hz, 2H), 1.67 (s, 3H), 1.54 - 1.43 (m, 4H), 1.27 (dt, *J* = 14.1, 7.2 Hz, 3H), 1.07 (q, *J* = 4.1 Hz, 1H), 1.02 (t, *J* = 7.0 Hz, 3H), 0.82 (t, *J* = 7.4 Hz, 3H), 0.55 (td, *J* = 8.2, 4.3 Hz, 1H).

Single crystal growth method for the final product 3 in Example 3: 20 mg of solid was weighed and placed in an ampoule filled with a mixed solution of 1 ml of ethyl acetate and 2 ml of n-hexane, and cotton was put at the bottle mouth to slow down the evaporation rate. The ampoule was allowed to stand still at room temperature to evaporate the solvent and a crystal was obtained 2 days later. The experimental parameters and results obtained by the single crystal X-ray diffraction analysis were as follows. After analysis, the configuration of compound 3 was as shown in FIG. 1, which was consistent with that of the final product 3 of Example 3.

| | | | | |
|---|---|---|---|---|
| Bond precision: | C-C = 0.0057 A | Wavelength=0.71073 | | |
| Cell: | a=11.1966(4) | b=19.7562(6) | | c=15.6339(5) |
| | alpha=90 | beta=102.384(1) | | gamma=90 |
| Temperature: | 150 K | | | |

| | Calculated | | Reported | |
|---|---|---|---|---|
| Volume | 3377.79(19) | | 3377.79(19) | |
| Space group | P 21/c | | P 1 21/c 1 | |
| Hall group | -P 2ybc | | -P 2ybc | |
| Moiety formula | C33 840 N4 O5 S | | C33 H40 N4 O5 S | |
| Sum formula | C33 840 N4 O5 S | | C33 840 N4 05 S | |
| Mr | 604.75 | | 604.75 | |
| Dx, g cm-3 | 1.189 | | 1.189 | |
| Z | 4 | | 4 | |
| Mu (mm-1) | 0.139 | | 0.139 | |
| FOOD | 1288.0 | | 1288.0 | |
| F000' | 1289.03 | | | |
| h, k, lmax | 14,25,20 | | 14,25,20 | |
| Nref | 7524 | | 7430 | |
| Tmin, Tmax | 0.959,0.969 | | 0.394,0.752 | |
| Tmin' | 0.959 | | | |
| Correction method= # Reported T Limits: Tmin=0.394 Tmax=0.752 AbsCorr = MULTI-SCAN | | | | |
| Data completeness= 0.988 | | Theta(max)= 27.185 | | |
| R(reflections)= 0.0800( 5509) | | | | wR2(reflections)= 0.2745( 7430) |
| S = 1.039 | Npar= 464 | | | |

### Example 4

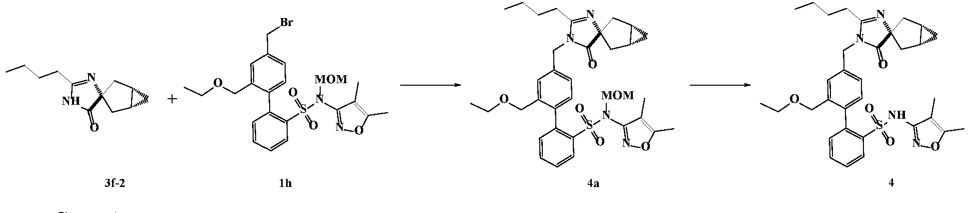

### Step1

Compound **3f-2** (50 mg, 0.24 mmol) was dissolved in N,N-dimethylformamide (2 mL). After cooling to 0°C, sodium hydride (60%, 12 mg, 0.31 mmol) was added and stirred for 30 min, and a solution of the compound **1h** (114 mg, 0.22 mmol) in N,N-dimethylformamide (0.5 mL) was added dropwise and continued to react for 5 hours. After the completion of the reaction was monitored by LC-MS, the system was poured into saturated ammonium chloride (10 mL), and extracted with ethyl acetate (3*10 mL). The organic phases were combined, washed with a saturated sodium chloride solution, dried over anhydrous sodium sulfate, and purified by column chromatography (petroleum ether:ethyl acetate=4:1) to afford compound 4a (85.0 mg, 0.13 mmol, yield: 54%). MS-ESI calc. for 649.3 [M+H]⁺, found 649.3.

### Step 2

Compound **4a** (85 mg, 0.13 mmol) was dissolved in a mixed solution of 95% ethanol (3 mL) and hydrochloric acid (6 **M**, 3 mL), and reacted at 75°C for 3 hours. LC-MS might detect production of 80% of the product. After the completion of the reaction, the pH value of the system was adjusted to 8 with 5 **M** sodium hydroxide solution, and then adjusted to 5 with 0.5 **M** dilute hydrochloric acid. The aqueous phase was extracted with ethyl acetate (20 mL), washed with saturated saline, dried over anhydrous sodium sulfate, and concentrated under reduced pressure. The crude product was purified by reversed-phase column chromatography (ammonium bicarbonate - acetonitrile system), and then purified by normal-phase column chromatography (petroleum ether:ethyl acetate=1:1) to afford compound **4** (53 mg, 0.09 mmol, yield: 69%). MS-ESI calc. for 605.3 [M+H]⁺, found 605.2. ¹H NMR (400 MHz, DMSO-*d*₆) *δ* 10.49 (s, 1H), 8.06 (dd, *J* = 8.1, 1.3 Hz, 1H), 7.63 (td, *J* = 7.4, 1.6 Hz, 2H), 7.19 (dd, *J* = 7.3, 1.7 Hz, 1H), 7.14 (d, *J* = 1.7 Hz, 1H), 6.98 (dd, *J* = 7.9, 1.8 Hz, 1H), 6.94 (d, *J* = 7.8 Hz, 1H), 4.73 (s, 2H), 4.00 (s, 2H), 3.28 - 3.13 (m, 2H), 2.33 (t, *J* = 7.4 Hz, 2H), 2.23 (s, 1H), 2.21 (s, 3H), 1.72 (d, *J* = 12.9 Hz, 2H), 1.67 (s, 3H), 1.54 - 1.44 (m, 4H), 1.32 - 1.21 (m, 3H), 1.12 (q, *J* = 4.1 Hz, 1H), 1.02 (t, *J* = 7.0 Hz, 3H), 0.82 (t, *J* = 7.3 Hz, 3H), 0.42 (td, *J* = 8.0, 4.1 Hz, 1H).

### Example 5

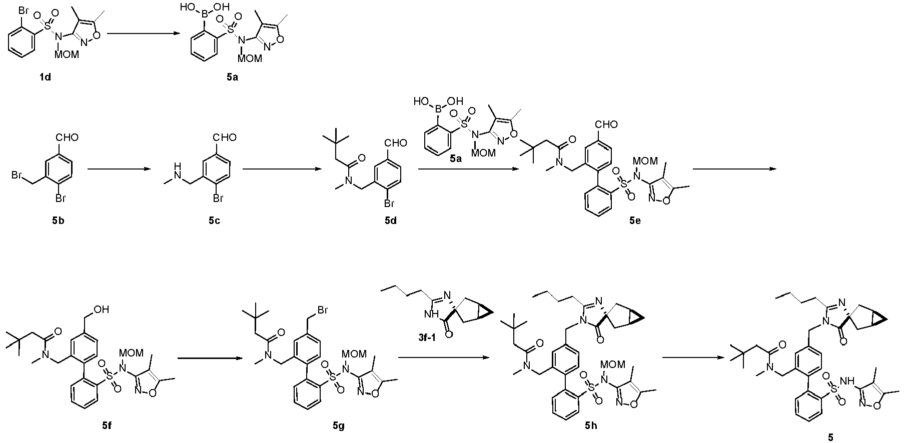

### Step 1

Compound **1d** (1.00 g, 2.66 mmol), bis(neopentyl glycolato)diboron (910 mg, 4.03 mmol), [1,1'-bis(diphenylphosphino)ferrocene]dichloropalladium - dichloromethane complex (110 mg, 0.14 mmol), and potassium acetate (520 mg, 5.31 mmol) were added to a three-necked flask successively. After a double-row tube was utilized to remove air and charge nitrogen gas, dioxane (20 mL) was added. The reaction system was heated and refluxed for 3 hours while stirring. After the completion of the reaction was monitored by LC-MS, the system was filtered by diatomite and then concentrated to dryness directly. The crude product was purified by reversed-phase medium-pressure preparative chromatography (water - acetonitrile system) to afford compound **5a** (360 mg, 1.06 mmol, yield: 40%). ¹H NMR (600 MHz, Chloroform-*d*) δ 7.86 (d, *J* = 8.0 Hz, 1H), 7.70 (d, *J* = 7.4 Hz, 1H), 7.60 (td, *J* = 7.5, 1.2 Hz, 1H), 7.51 (td, *J* = 7.7, 1.5 Hz, 1H), 5.02 (s, 2H), 3.25 (s, 3H), 2.36 (s, 3H), 1.98 (s, 3H).

### Step 2

Compound **5b** (3.00 g, 10.79 mmol) was dissolved in anhydrous tetrahydrofuran (30 mL), and a methylamine tetrahydrofuran solution (2**M**, 50 mL, 100 mmol) was added to the system and stirred at room temperature for 30 min. After the completion of the reaction was monitored by LC-MS, the reaction mixture was poured into water (80 mL), and extracted with ethyl acetate (160 mL*3). The organic phases were combined, washed with saturated saline (80 mL), dried over anhydrous sodium sulfate, and then concentrated under reduced pressure to obtain a crude product of compound **5c** (2.50 g, 10.96 mmol, yield of crude product: 102%). MS-ESI calc. for 228.0 [M+H]⁺, found 228.2.

### Step 3

Compound **5c** (2.50 g, 10.96 mmol) was dissolved in anhydrous tetrahydrofuran (80 mL) and cooled to 0°C, and triethylamine (6.80 g, 67.20 mmol) and 3,3-dimethylbutyryl chloride (3.00 g, 22.29 mmol) were added in sequence, held and stirred for 30 min. After the completion of the reaction was monitored by LC-MS, the reaction solution was poured into ice water (80 mL), and extracted with ethyl acetate (160 mL). The organic phase was separated, washed with saturated saline (80 mL), dried over anhydrous sodium sulfate, and then distilled under reduced pressure. The resulting crude product was purified by column chromatography (petroleum ether:ethyl acetate=3:2) to afford compound **5d** (2.70 g, 8.28 mmol, yield: 76%). MS-ESI calc. for 326.1 [M+H]⁺, found 325.8.

### Step 4

Compound **5d** (500 mg, 1.53 mmol) and compound **5a** (690 mg, 2.03 mmol) were dissolved in a mixed solution of toluene (10 mL) and water (4 mL) and ethanol (5 mL). After nitrogen gas was charged in the system and bubbled for 10 min, tetrakis(triphenylphosphine)palladium (90.0 mg, 0.08 mmol) and sodium carbonate (500 mg, 4.72 mol) were added successively. The system was stirred at 85°C for 3 hours. After the completion of the reaction was monitored by LC-MS, the reaction system was extracted with ethyl acetate (20 mL*3). The organic phases were combined, washed with saturated saline (10 mL), dried over anhydrous sodium sulfate, and concentrated under reduced pressure. The resulting crude product was purified by column chromatography (petroleum ether: ethyl acetate=2.5:1) to afford compound **5e** (102 mg, 0.19 mmol, yield: 12%). MS-ESI calc. for 542.2 [M+H]⁺, found 542.1.

### Step 5

Compound **5e** (102 mg, 0.19 mmol) was dissolved in methanol (5 mL) and cooled to -40°C, and sodium borohydride (5.0 mg, 0.13 mmol) was added and stirred for 1 hour while maintaining the temperature. After the completion of the reaction was monitored by LC-MS, the system was poured into dilute hydrochloric acid (0.5 **M**, 15 ml), and extracted with ethyl acetate (20 mL*3). The organic phases were combined, washed with saturated saline (20 mL), dried over anhydrous sodium sulfate, and then concentrated under reduced pressure. The resulting crude product was purified by column chromatography (petroleum ether: ethyl acetate=4:1) to afford compound **5f** (91 mg, 0.17 mmol, yield: 89%). MS-ESI calc. for 544.2 [M+H]⁺, found 544.2.

### Step 6

At 0°C, compound **5f** (91 mg, 0.17 mmol) was dissolved in anhydrous N,N-dimethylformamide (5 mL), and tetrabromomethane (169 mg, 0.51 mmol) and triphenylphosphine (134 mg, 0.51 mmol) were added successively, and stirred for 2 hours while maintaining the temperature. After the completion of the reaction was monitored by LC-MS, the system was concentrated under reduced pressure. The resulting crude product was purified by column chromatography (petroleum ether:ethyl acetate=9:1) to afford compound **5g** (75 mg, 0.12 mmol, yield: 71%). MS-ESI calc. for 606.2 [M+H]⁺, found 606.1.

### Step 7

At 0°C, compound **3f-1** (24.0 mg, 0.12 mmol) was dissolved in anhydrous N,N-dimethylformamide (1 mL). Sodium hydride (60%, 10.6 mg, 0.26 mmol) was added and stirred for 30 min. Afterwards, a solution of compound **5g** (70 mg, 0.12 mmol) in N,N-dimethylformamide (0.5 mL) was added to the system and stirred at room temperature for 3 hours. After the completion of the reaction was monitored by LC-MS, the system was poured into saturated ammonium chloride (10 mL), and extracted with ethyl acetate (20 mL). The organic phase was separated, washed with saturated saline (10 mL), dried over anhydrous sodium sulfate, and then distilled under reduced pressure. The resulting crude product was purified by column chromatography (petroleum ether:ethyl acetate=4:1) to afford compound **5h** (65 mg, 0.09 mmol, yield: 75%). MS-ESI calc. for 732.4 [M+H]⁺, found 732.4.

### Step 8

Compound **5h** (60 mg, 0.08 mmol) was dissolved in a mixed solution of ethanol (6 mL) and hydrochloric acid (6 **M**, 5 mL), and stirred at 75°C for 3 hours. After the completion of the reaction was monitored by LC-MS, the pH value of the system was adjusted to 8 with sodium hydroxide solution (5 **M**), and then adjusted to 5 with dilute hydrochloric acid (0.5 **M**). The reaction system was extracted with ethyl acetate (20 mL*3). The organic phases were combined, washed with saturated saline (20 mL), dried over anhydrous sodium sulfate, and then concentrated under reduced pressure. The resulting crude product was prepared by the preparative chromatography (water - acetonitrile) system to afford compound **5** (30 mg, 0.04 mmol, yield: 50%, HPLC purity: 98.2%). MS-ESI calc. for 688.4 [M+H]⁺, found 688.4. ¹H NMR (600 MHz, DMSO-*d*₆) δ 10.63 (s, 1H), 8.13 - 7.98 (m, 1H), 7.76 - 7.54 (m, 2H), 7.24 (t, *J* = 8.1 Hz, 1H), 7.12 - 6.88 (m, 2H), 6.83 - 6.65 (m, 1H), 4.78 - 4.54 (m, 2H), 4.40 - 3.99 (m, 2H), 2.80 - 2.59 (m, 3H), 2.35 - 2.10 (m, 8H), 1.97 - 1.87 (m, 1H), 1.87 - 1.79 (m, 2H), 1.77 - 1.68 (m, 3H), 1.59 - 1.37 (m, 4H), 1.32 - 1.17 (m, 2H), 1.14 - 1.02 (m, 1H), 1.01 - 0.75 (m, 12H), 0.61 - 0.48 (m, 1H).

### Example 6

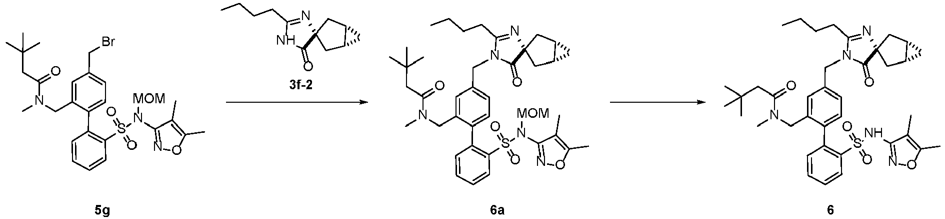

### Step 1

At 0°C, compound **3f-2** (26.0 mg, 0.13 mmol) was dissolved in anhydrous N,N-dimethylformamide (1.2 mL), and sodium hydride (60%, 11.2 mg, 0.28 mmol) was added and stirred for 30 min. Thereafter, a solution of compound **5g** (75 mg, 0.12 mmol) in N,N-dimethylformamide (0.7 mL) was added to the system, and stirred at room temperature for 3 hours. After the completion of the reaction was monitored by LC-MS, the system was poured into saturated ammonium chloride (10 mL), and extracted with ethyl acetate (20 mL). The organic phase was separated, washed with saturated saline (10 mL), dried over anhydrous sodium sulfate, and then distilled under reduced pressure. The resulting crude product was purified by column chromatography (petroleum ether:ethyl acetate=4:1) to afford compound **6a** (67 mg, 0.09 mmol, yield: 69%). MS-ESI calc. for 732.4 [M+H]⁺, found 732.3.

### Step 2

Compound **6a** (65 mg, 0.09 mmol) was dissolved in a mixed solution of ethanol (5 mL) and hydrochloric acid (6 **M**, 5 mL), and stirred at 75°C for 3 hours. After the completion of the reaction was monitored by LC-MS, the pH value of the system was adjusted to 8 with a sodium hydroxide solution (5 **M**), and then adjusted to 5 with dilute hydrochloric acid (0.5 **M**). The reaction system was extracted with ethyl acetate (20 mL*3). The organic phases were combined, washed with saturated saline (20 mL), dried over anhydrous sodium sulfate, and then concentrated under reduced pressure. The resulting crude product was prepared by the preparative chromatography (ammonium bicarbonate: acetonitrile) system to afford compound **6** (37 mg, 0.05 mmol, yield: 56%, HPLC purity: 99.0%). MS-ESI calc. for 688.4 [M+H]⁺, found 688.4. ¹H NMR (600 MHz, Methanol-*d*₄) δ 8.35 - 7.98 (m, 1H), 7.73 - 7.39 (m, 2H), 7.23 - 7.10 (m, 1H), 7.10 - 6.96 (m, 2H), 6.94 - 6.72 (m, 1H), 4.82 - 4.70 (m, 2H), 4.62 - 4.14 (m, 2H), 2.95 - 2.72 (m, 3H), 2.51 - 2.24 (m, 5H), 2.24 - 1.93 (m, 4H), 1.87 - 1.77 (m, 2H), 1.75 - 1.66 (m, 3H), 1.61 - 1.46 (m, 4H), 1.42 - 1.25 (m, 2H), 1.11 - 0.80 (m, 13H), 0.61 - 0.33 (m, 1H).

### Example 7

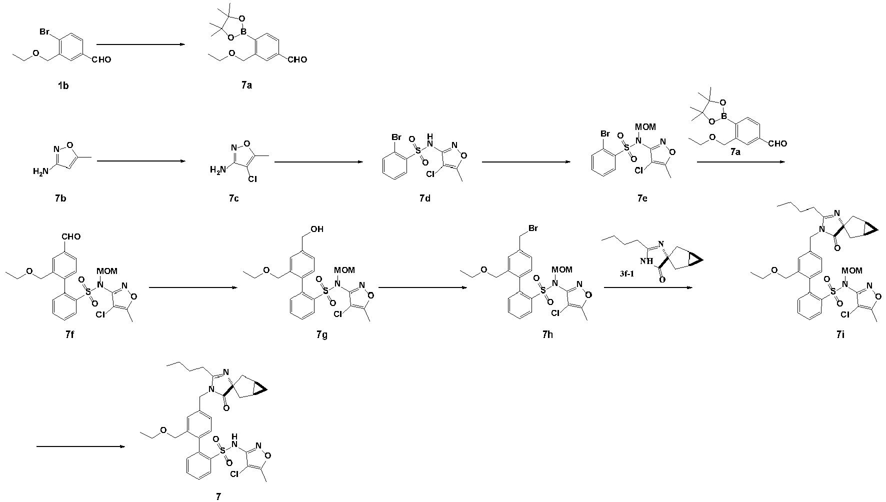

### Step 1

Under the protection of nitrogen gas, compound **1b** (30 g, 0.12 mol), bis(pinacolato)diboron (32.9 g, 0.13 mol), potassium acetate (24.2 g, 0.25 mol), and [1,1'-bis(diphenylphosphino)ferrocene]dichloropalladium - dichloromethane complex (5.0 g, 6.22 mmol) were added to a dioxane solution (800 mL) successively, and refluxed for 16 hours. After the completion of the reaction was monitored by TLC, the resultant was filtered by diatomite to remove the solid, and concentrated under reduced pressure to remove dioxane. The resultant was transferred into water (400 mL), and then extracted with ethyl acetate (300 mL*3). The organic phases were combined, washed with saturated saline (300 mL*3), dried over anhydrous sodium sulfate, and concentrated under reduced pressure to obtain a crude product of compound **7a** (56 g).

### Step 2

Under the protection of nitrogen gas, compound **7b** (980 mg, 10.00 mmol) was dissolved in dichloromethane (15 mL) and cooled to 0°C, and N-chlorosuccinimide (1.46 g, 10.93 mmol) was added slowly to this system and continued to react for 2 hours at this temperature. A sodium hydroxide solution (**1 M**, 10 mL) was added to quench the reaction. The aqueous phase was extracted with dichloromethane (15 mL). The organic phase was combined, washed with saturated saline (50 mL), dried over anhydrous sodium sulfate, and concentrated under reduced pressure. The resulting crude product was purified by column chromatography (petroleum ether:ethyl acetate= 5:1) to afford compound **7c** (712 mg, 5.37 mmol, yield: 54%). MS-ESI [M+ H]⁺, found 133.1.

### Step 3

At 0°C, compound **7c** (500 mg, 3.77 mmol), 2-bromobenzenesulfonyl chloride (962 mg, 3.76 mmol), and 4-dimethylaminopyridine (62.0 mg, 0.51 mmol) were added to pyridine (15 mL) successively, stirred for 10 min, and heated to 40°C and reacted for 12 hours. After the completion of the reaction was detected by LC-MS, the system was transferred to an ice bath, adjusted to pH 6 with iced hydrochloric acid (**1 M**), and extracted with ethyl acetate (15 mL*3). The organic phases were combined, washed with saturated saline (50 mL), dried over anhydrous sodium sulfate, and concentrated under reduced pressure to afford compound **7d** (1.3 g, 3.70 mmol, yield: 98%). MS-ESI [M+ H]⁺, found 351.1.

### Step 4

At -15°C, compound **7d** (1.3 g, 3.70 mmol) was dissolved in N, N-dimethylformamide (10 mL), and sodium hydride (60%, 171 mg, 4.28 mmol) was added in batches, stirred for 5 min and then heated to room temperature and reacted for 30 min. Thereafter, the system was transferred to -15°C. Bromomethyl methyl ether (600 mg, 4.80 mmol) was added dropwise, stirred for 5 min, and then naturally warmed and reacted for 30 min. After the completion of the reaction was detected by LC-MS, the reaction system was transferred to an ice bath, quenched by adding cold water (40 mL), adjusted to pH 7 with hydrochloric acid (**0.5 M**), and extracted with ethyl acetate (15 mL*3). The organic phases were combined, washed with saturated saline (50 mL), dried over anhydrous sodium sulfate, and concentrated under reduced pressure. The resulting crude product was purified by column chromatography (petroleum ether:ethyl acetate= 8:1) to afford compound **7e (**777 mg, 1.96 mmol, yield: 53%). MS-ESI [M+ H]⁺, found 394.9.

### Step 5

Under the protection of nitrogen gas, compound **7e** (300 mg, 0.76 mmol), compound **7a** (220 mg, 0.76 mmol), tetrakis(triphenylphosphine)palladium (114 mg, 0.099 mmol), and sodium carbonate (402 mg, 3.79 mmol) were added to a mixed solution of toluene (15 mL), water (15 mL) and ethanol (15 mL), successively. The system was reacted at 85°C for 2 hours. After the completion of the reaction was detected by LC-MS, the resultant was filtered by diatomite, and concentrated under reduced pressure. The resulting crude product was purified by column chromatography (petroleum ether: ethyl acetate= 5:1) to afford compound **7f** (255 mg, 0.53 mmol, yield: 70%). MS-ESI [M+ H]⁺, found 479.0.

### Step 6

At 0°C, compound **7f** (235 mg, 0.49 mmol) was dissolved in methanol (5 mL), and sodium borohydride (29.8 mg, 0.79 mmol) was added. After 5 min, the system was transferred to 25°C and reacted for 1 hour. The system was transferred to an ice bath, added with ice water (30 mL), adjusted to pH=7 with hydrochloric acid (**1 M**), and extracted with ethyl acetate (20 mL*3). The organic phases were combined, washed with saturated saline (100 mL), dried over anhydrous sodium sulfate, and concentrated under reduced pressure to obtain a crude product **7g** (237 mg), which was used directly in the next reaction step. MS-ESI [M+ H]⁺, found 481.0.

### Step 7

At 0°C, compound **7g** (237 mg), tetrabromomethane (243 mg, 0.73 mmol), and triphenylphosphine (194 mg, 0.74 mmol) were added to anhydrous N, N-dimethylformamide (5 mL) successively, and continued to react for 2 hours while maintaining this temperature. After the complete reaction of raw materials was detected by LC-MS, ice water (30 mL) was added to the system, and the system was extracted with ethyl acetate (20 mL*3). The organic phases were combined, washed with saturated saline (80 mL), dried over anhydrous sodium sulfate, and concentrated under pressure. The resulting crude product was purified by column chromatography (petroleum ether:ethyl acetate= 3:1) to afford compound **7h** (172 mg, 0.32 mmol, two-step yield: 65%). MS-ESI [M+ H]⁺, found 543.3.

### Step 8

At 0°C, compound **3f-1** (25.0 mg, 0.12 mmol) was dissolved in anhydrous N,N-dimethylformamide (2 mL), and sodium hydride (60%, 5.34 mg, 0.13 mmol) was added, stirred for 5 min, then heated to room temperature, and stirred for 30 min. Thereafter, the system was transferred to an ice bath. A solution of compound **7h** (55 mg, 0.10 mmol) dissolved in N,N-dimethylformamide (2 mL) was slowly added dropwise to the reaction system. After 5 min, the reaction system was heated to room temperature and reacted for 1 hour. After the complete reaction of raw materials was detected by LC-MS, ice water (30 mL) was added to the system, and the system was adjusted to pH=7 with hydrochloric acid (**1 M**), and extracted with ethyl acetate (3*10 mL). The organic phases were combined, washed with saturated saline (50 mL), dried over anhydrous sodium sulfate, and distilled under reduced pressure. The resulting crude product was purified by column chromatography (petroleum ether:ethyl acetate= 2:1) to afford compound **7i** (67.0 mg, 0.10 mmol, yield: 100%). MS-ESI [M+ H]⁺, found 669.1.

### Step 9

Compound **7i** (67.0 mg, 0.10 mmol) was dissolved in anhydrous ethanol (2.5 mL), and hydrochloric acid (**6 N**, 2.5 mL) was added and heated at 40°C for 1 hour. After the completion of the reaction was detected by LC-MS, the system was transferred to an ice bath, adjusted to pH=7 with a sodium bicarbonate solid, and extracted with ethyl acetate (10 mL*3). The organic phases were combined, washed with saturated saline (50 mL), dried over anhydrous sodium sulfate, and purified by reversed-phase chromatography (ammonium bicarbonate - acetonitrile system) to afford compound **7** (31 mg, 0.05 mmol, yield: 50%). MS-ESI [M+H]⁺, found 625.2. ¹H NMR (600 MHz, Chloroform-*d*) δ 8.28 (dd, *J* = 8.0, 1.4 Hz, 1H), 7.62 (td, *J* = 7.5, 1.4 Hz, 1H), 7.57 (td, *J* = 7.8, 1.5 Hz, 1H), 7.35 (d, *J* = 2.0 Hz, 1H), 7.25 (dd, *J* = 7.5, 1.4 Hz, 1H), 7.10 (d, *J* = 7.7 Hz, 1H), 7.05 (dd, *J* = 7.8, 1.9 Hz, 1H), 4.69 (d, *J* = 2.3 Hz, 2H), 4.16 (d, *J* = 12.4 Hz, 1H), 4.08 (d, *J* = 12.3 Hz, 1H), 3.33 (ttd, *J* = 9.4, 7.0, 2.3 Hz, 2H), 2.34 (d, *J* = 9.0 Hz, 6H), 2.31 (d, *J* = 4.0 Hz, 1H), 1.98 (dd, *J* = 13.8, 2.4 Hz, 2H), 1.65 (tt, *J* = 9.1, 6.9 Hz, 2H), 1.58 - 1.52 (m, 2H), 1.42 - 1.31 (m, 2H), 1.09 (t, *J* = 7.0 Hz, 3H), 1.06 (q, *J* = 4.3 Hz, 1H), 0.91 (t, *J* = 7.4 Hz, 3H), 0.66 (td, *J* = 8.2, 4.7 Hz, 1H).

The technical effects of the compounds of the present disclosure will be described below with reference to the Experimental Examples.

### Experimental Example 1. Calcium flux assay on AT1 cell line

Test materials: a) cell line: AT1/HEK293; b) vehicles: F12, Invitrogen (Cat#11765-047); FBS, Corning (Cat# 35-076-CV); Geneticin, Invitrogen (Cat# 10131); c) reagent: Fluo-4 Direct, (Invitrogen, Cat# F10471); d) instruments: 384 well Poly-D-Lysine protein coating plate, Greiner #781946; Vi-cell XR Cell Viability Analyzer, Beckman Coulter; Incubator, Thermo.

### Test method:

For the agonist (angiotensin II) master plate, the agonist solution was diluted from 1 mM to 200 µM with the test buffer solution. For the antagonist (Losartan) plates and the compound plates, the antagonist and the tested compounds were diluted with DMSO at a gradient ratio of 1:4 to obtain 10 concentration points. Afterwards, 900 nL of each compound solution was transferred to the master plate and 30 µL of the test buffer solution was added.
a) The cell plate was removed from the incubator, and 20 µL of 2X Fluo-4 Direct was added by a pipette to a 384-well cell plate.
b) The cell plate was incubated at 37°C for 50 min in the environment of 5% CO₂, and then incubated at room temperature for 10 min.
c) The cell plate was removed from the incubator and placed in FLIPR. The compound plates and the tip box were placed in FLIPR.
d) Regarding the EC80 plate:
   1) Operations were conducted on FLIPRTETRA.
   2) 10 µL of compound solution was transferred from the EC80 master plate to the cell plate.
   3) Fluorescence signals were read.
   4) The read signals were counted, and the EC80 value of each cell line was calculated using FLIPR.
   5) For the concentration of the compound plate, a solution having a concentration 6 times that of the reference agonist EC80 was formulated.
e) Regarding the compound plate:
   1) Operations were conducted on FLIPRTETRA.
   2) 10 µL of compound was transferred to the cell plate.
   3) Fluorescence signals were read.
   4) 10 µL of solution having a concentration 6 times that of the reference agonist EC80 was transferred.
   5) Fluorescence signals were read.
f) Data analysis by Prism

**Table 1 (IC₅₀ activity against AT1: + denoting 100 nM to 1 µM, ++ denoting 10 nM to 100 nM, +++ denoting 1 nM to 10 nM, the specific activities were as shown in the third column of Table 1)**

| Compound Name | IC₅₀ (nM) | IC₅₀ (nM) |
|---|---|---|
| Example 1 | +++ | 2.90 nM |
| Example 2 | +++ | 6.14 nM |
| Example 3 | +++ | 1.97 nM |
| Example 4 | +++ | 4.78 nM |
| Example 5 | ++ | 26.82 nM |
| Example 6 | + | 257.6 nM |
| Example 7 | +++ | 3.55 nM |
| Sparsentan | +++ | 9.79 nM |
| Losartan | +++ | 1.86 nM |

### Experimental Example 2. Calcium flux assay on ETa cell line

Test materials: a) cell line: ETa/HEK293; b) vehicles: DMEM, Invitrogen (Cat#11960); Geneticin, Invitrogen (Cat# 10131); c) reagent: Fluo-4 Direct, (Invitrogen, Cat# F10471); d) instruments: 384 well Poly-D-Lysine protein coating plate, Greiner #781946; Vi-cell XR Cell Viability Analyzer, Beckman Coulter; Incubator, Thermo.

### Test method:

For the employed agonist endothelin factor (endothelin-1, ET-1) master plate: the agonist was diluted from 50 µM to 15 µM with the test buffer solution.

For antagonist (BQ123, Am J Physiol. 1994 Apr; 266(4 Pt 2): H1327-31.) plates and the compound plates: the antagonist and the tested compounds were diluted with DMSO at a gradient ratio of 1:4 to obtain 10 concentration points. Afterwards, 900 nL of each compound solution was transferred to the master plate and 30 µL of the test buffer solution was added.
a) The cell line was removed from the incubator, and 20 µL of 2X Fluo-4 Direct^{™} buffer solution was added gently by a pipette to a 384-well cell culture plate.
b) The cell plate was incubated at 37°C for 50 min in the environment of 5% CO₂, and then incubated at room temperature for 10 min.
c) The cell plate was removed from the incubator and placed in FLIPR. The compound plates and the tip box were placed in FLIPR.
d) Regarding the EC80 plate:
   1) Operations were conducted on FLIPRTETRA.
   2) 10 µL of compound solution was transferred from the EC80 master plate to the cell plate.
   3) Fluorescence signals were read.
   4) The read signals were counted, and the EC80 value of each cell line was calculated using FLIPR.
   5) For the concentration of the compound plate, a solution having a concentration 6 times that of the reference agonist EC80 was formulated.
e) Regarding the compound plate:
   1) Operations were conducted on FLIPRTETRA.
   2) 10 µL of compound was transferred to the cell plate.
   3) Fluorescence signals were read.
   4) 10 µL of solution having a concentration 6 times that of the reference agonist EC80 was transferred to the cell plate.
   5) Fluorescence signals were read.
f) Data analysis by Prism

**Table 2 (IC₅₀ activity against ETa: + denoting 100 nM to 1 µM, ++ denoting 10 nM to 100 nM, +++ denoting 1 nM to 10 nM, the specific activities were as shown in the third column of Table 2)**

| Compound Name | IC₅₀ (nM) | IC₅₀ (nM) |
|---|---|---|
| Example 1 | ++ | 24.60 nM |
| Example 2 | ++ | 23.55 nM |
| Example 3 | ++ | 10.53 nM |
| Example 4 | + | 131.60 nM |
| Example 5 | ++ | 14.83 nM |
| Example 6 | ++ | 17.58 nM |
| Example 7 | ++ | 24.29 nM |
| Sparsentan | ++ | 80.81 nM |
| BQ123 | +++ | 2.56 nM |

### Experimental Example 3. Experiment on water solubility

The detection was carried out in accordance with the high performance liquid chromatography (General Principles 0512 of Chinese Pharmacopoeia Volume IV (2020*)*):
Chromatographic conditions for the HPLC-UV method: mobile phase: 0.02 M KH₂PO₄ aqueous solution-acetonitrile (90:10), gradient elution; chromatographic column: C₁₈; detection wavelength: 210 nm. The water solubility was calculated based on the peak area by the external standard method.

**Table 3**

| Compound Name | Solubility (mg/mL) | | | |
|---|---|---|---|---|
| | FaSSGF (pH1.6) | FeSSIF (pH5.0) | FaSSIF (pH6.5) | pH7.4 |
| Example 1 | > 1 | 0.20 | 0.61 | 0.37 |
| Example 2 | 0.03 | 0.27 | 0.35 | 0.86 |
| Example 3 | > 1 | 0.06 | 0.19 | 0.82 |
| Example 5 | 0.12 | 0.06 | 0.19 | 0.41 |
| Example 7 | 0.24 | 0.08 | 0.87 | 0.84 |
| Sparsentan | 0.13 | 0.02 | 0.08 | 0.21 |

Conclusion: Most of the specific compounds prepared in the present disclosure had better water solubility than that of the reference compound under multiple test conditions.

### Experimental Example 4. Inhibitory effects on activities of cytochrome P450 isozymes (CYP1A2, CYP2C9, CYP2C19, CYP2D6, and CYP3A4) from human liver microsomes

A total of six specific probe substrates of six isozymes of CYPs, i.e., α-Naphthoflavone (CYP1A2), Sulphaphenazole (CYP2C9), Ticlopidine (CYP2C19), Quinidine (CYP2D6), Ketoconazole (CYP3A4), and Montelukast (CYP2C8) were co-incubated with recombinant hepatic drug enzymes CYP1A2, CYP2C9, CYP2C19, CYP2D6, CYP3A4, and CYP2C8 and test compounds, respectively. Nicotinamide adenine dinucleotide phosphate (NADP+), D-glucose-6-phosphate (G6P) and glucose-6-phosphate dehydrogenase (G6DHP) were added to start the reaction. At the end of the reaction, the corresponding half maximal inhibitory concentrations (IC₅₀) were calculated by the fluorescence detection method (Ex490nm/Em520nm).

**Table 4**

| Compound No. | IC₅₀ (µM) | | | | | |
|---|---|---|---|---|---|---|
| | CYP1A2 | CYP2C9 | CYP2C19 | CYP2D6 | CYP3A4 | CYP2C8 |
| Example 3 | >10 | >10 | >10 | >10 | 1<IC₅₀<10 | >10 |
| Example 7 | >10 | >10 | >10 | >10 | >10 | >10 |
| Sparsentan | >10 | >10 | >10 | >10 | 1<IC₅₀<10 | 1<IC₅₀<10 |

Conclusion: Sparsentan had no risk of inhibiting the activities of cytochrome P450 isozymes CYP1A2, CYP2C9, CYP2C19, and CYP2D6 from human liver microsomes, but had an inhibitory effect on CYP3A4 and CYP2C8. The compound of Example 3 had no risk of inhibiting the activities of cytochrome P450 isozymes CYP1A2, CYP2C9, CYP2C19, CYP2D6 and CYP2C8, and it had an inhibitory effect only on CYP3A4. The compound of Example 7 had no risk of inhibiting the activities of all cytochrome P450 isozymes CYP1A2, CYP2C9, CYP2C19, CYP2D6, CYP3A4, and CYP2C8.

The above data anticipated a lower risk of DDI (drug-drug interaction) for clinical use of the compounds of Example 3 and Example 7.

### Experimental Example 5. Pharmacokinetic test in rats

Experimental animal: SD rats, 6-8 weeks. Six animals were used for each example compound.

Drug solution formulation: all of the Experimental Example compounds were formulated into 1 mg/mL solution. The solution system was 5%DMSO + 20%Solutol HS15 + 75%(20%HP-β-CD aqueous solution).

Grouping for administration: the first group was administered by oral gavage (PO, three SD rats, 10 mg/kg, fasted overnight before administration, and fed after 4 hours following the administration); and the second group was administered by intravenous injection in dorsum of foot (IV, three SD rats, 1 mg/kg, fed freely).

Blood sampling time and treatment method: Blood was collected via the jugular vein at the following 7 to 8 points in time: 0.083 (only in the intravenous injection group), 0.25, 0.5, 1, 2, 4, 8, and 24 hours. The blood samples were centrifuged (2000 g, 4°C, 5 min) to obtain plasma. The samples were stored at -70°C before analysis.

Analytical instruments and conditions: LC-MS/MS-33 (Triple Quad 6500+); MS: positive, ESI; mobile phases: Mobile phase A: H2O-0.025%FA-1mM NH₄OAc, Mobile phase B: MeOH-0.025%FA-1mM NH₄OAc; flow rate: 0.60 mL/min; chromatographic column: ACQUITY UPLC-BEH C₁₈ (2.1×50 mm, 1.7 µm); column temperature: 60°C.

**Table 5: Pharmacokinetic Parameters for Oral Administration (PO)**

| | Tₘₐₓ (hr) | Cₘₐₓ (ng/mL) | T_{1/2} (hr) | AUCₗₐₛₜ (hr*ng/mL) | Bioavailability (%) |
|---|---|---|---|---|---|
| Example 3 (PO) | 2.17 | 2993 | 7.00 | 24407 | 69.3 |
| Example 7 (PO) | 3.33 | 3080 | 5.28 | 23530 | 43.4 |
| Sparsentan (PO) | 1.42 | 1803 | 15.4 | 18612 | 20.6 |

Conclusion: the oral gavage administration group showed that after oral administration, the compounds of Example 3 and Example 7 of the present application could achieve high *in vivo* exposure and higher oral bioavailability, and showed that the compounds of Example 3 and Example 7 had better pharmacokinetic properties than those of the reference compound.

The foregoing is only preferred embodiments of the present disclosure, but the scope of protection for the present disclosure is not limited to the above embodiments. Any equivalent modifications or variations made by one of ordinary skill in the art based on the contents disclosed herein shall be encompassed within the scope of protection disclosed in the claims.

## Claims

1. A compound represented by formula I, or a deuterated compound thereof, or a stereoisomer thereof, or a pharmaceutically acceptable salt thereof: 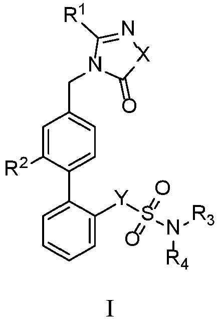 wherein
X is NR^{X1} or CR^{X2}R^{X3};
Y is a chemical bond, NR^{Y1} or CR^{Y2}R^{Y3};
R^{Y1} is selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring);
R^{Y2} and R^{Y3} are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring);
R^{X1} is selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring); wherein the alkylene, carbocyclyl, heterocycloalkyl, aromatic ring, or heteroaromatic ring is optionally further substituted by one, two, three or four independent R^{X11};
each R^{X11} is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR^{X12}, -C₀₋₄ alkylene-SR^{X12}, and -C₀₋₄ alkylene-N^{RX12}R^{X13}; or two independent R^{X11}, together with the atom to which they are linked directly, form 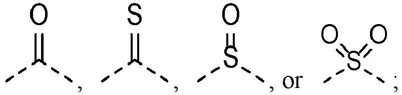
R^{X12} and R^{X13} are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen;
R^{X2} and R^{X3}, together with the atom to which they are linked directly, form a 6- to 12-membered spirocyclic ring, a 6- to 12-membered spiroheterocyclic ring, a 6- to 12-membered fused cyclic ring, or a 6- to 12-membered fused heterocyclic ring; wherein the spirocyclic ring, spiroheterocyclic ring, fused cyclic ring, or fused heterocyclic ring is optionally further substituted by one, two, three or four independent R^{X21};
each R^{X21} is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR^{X22}, -C₀₋₄ alkylene-SR^{X22}, and -C₀₋₄ alkylene-NR^{X22}R^{X23}; or two independent R^{X21}, together with the atom to which they are linked directly, form 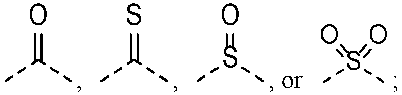
R^{X22} and R^{X23} are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen;
R¹ is selected from the group consisting of -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen;
R² is selected from the group consisting of -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR²¹, -C₀₋₄ alkylene-SR²¹, -C₀₋₄ alkylene-NR²¹R²², -C₀₋₄ alkylene-OC(O)R²¹, -C₀₋₄ alkylene-S(O)₂R²¹, -C₀₋₄ alkylene-S(O)R²¹, -C₀₋₄ alkylene-S(O)₂NR²¹R²², -C₀₋₄ alkylene-S(O)NR²¹R²², -C₀₋₄ alkylene-C(O)R²¹, -C₀₋₄ alkylene-C(O)OR²¹, -C₀₋₄ alkylene-C(O)NR²¹R²², -C₀₋₄ alkylene-NR²¹C(O)R²², -C₀₋₄ alkylene-NR²¹S(O)₂R²², -C₀₋₄ alkylene-NR²¹S(O)R²², -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring);
R²¹ and R²² are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring);
R³ and R⁴ are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring); wherein the alkylene, carbocyclyl, heterocycloalkyl, aromatic ring, or heteroaromatic ring is optionally further substituted by one, two, three or four independent R³¹;
each R³¹ is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₄ alkylene-OR³², -C₀₋₄ alkylene-SR³², -C₀₋₄ alkylene-NR³²R³³, -C₀₋₄ alkylene-OC(O)R³², -C₀₋₄ alkylene-S(O)₂R³², -C₀₋₄ alkylene-S(O)R³², -C₀₋₄ alkylene-S(O)₂NR³²R³³, -C₀₋₄ alkylene-S(O)NR³²R³³, -C₀₋₄ alkylene-C(O)R³², -C₀₋₄ alkylene-C(O)OR³², -C₀₋₄ alkylene-C(O)NR³²R³³, -C₀₋₄ alkylene-NR³²C(O)R³³, -C₀₋₄ alkylene-NR³²S(O)₂R³³, -C₀₋₄ alkylene-NR³²S(O)R³³, -C₀₋₄ alkylene-(3- to 10-membered carbocyclyl), -C₀₋₄ alkylene-(4- to 10-membered heterocycloalkyl), -C₀₋₄ alkylene-(6- to 10-membered aromatic ring), and -C₀₋₄ alkylene-(5- to 10-membered heteroaromatic ring); or two independent R³¹, together with the atom to which they are linked directly, form 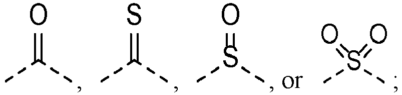
R³² and R³³ are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen.

2. The compound, or the deuterated compound thereof, or the stereoisomer thereof, or the pharmaceutically acceptable salt thereof according to claim 1, wherein
R¹ is n-butyl.

3. The compound, or the deuterated compound thereof, or the stereoisomer thereof, or the pharmaceutically acceptable salt thereof according to claim 1, wherein
R² is selected from the group consisting of -C₁₋₂ alkylene-OR²¹, -C₁₋₂ alkylene-SR²¹, -C₁₋₂ alkylene-NR²¹R²², -C₁₋₂ alkylene-OC(O)R²¹, -C₁₋₂ alkylene-S(O)₂R²¹, -C₁₋₂ alkylene-S(O)R²¹, -C₁₋₂ alkylene-S(O)₂NR²¹R²², -C₁₋₂ alkylene-S(O)NR²¹R²², -C₁₋₂ alkylene-C(O)R²¹, -C₁₋₂ alkylene-C(O)OR²¹, -C₁₋₂ alkylene-C(O)NR²¹R²², -C₁₋₂ alkylene-NR²¹C(O)R²², -C₁₋₂ alkylene-NR²¹S(O)₂R²², and -C₁₋₂ alkylene-NR²¹S(O)R²²;
R²¹ and R²² are each independently selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₂ alkylene-(3- to 10-membered carbocyclyl), and -C₀₋₂ alkylene-(4- to 10-membered heterocycloalkyl).

4. The compound, or the deuterated compound thereof, or the stereoisomer thereof, or the pharmaceutically acceptable salt thereof according to claim 3, wherein
R² is selected from the group consisting of 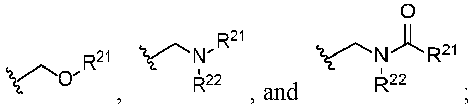
R²¹ and R²² are each independently selected from the group consisting of hydrogen, -C₁₋₆ alkyl, -C₁₋₆ alkyl substituted with halogen, and -C₀₋₂ alkylene-(3-6-membered carbocyclyl).

5. The compound, or the deuterated compound thereof, or the stereoisomer thereof, or the pharmaceutically acceptable salt thereof according to claim 4, wherein the R² is selected from the group consisting of

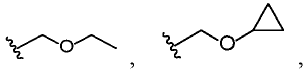

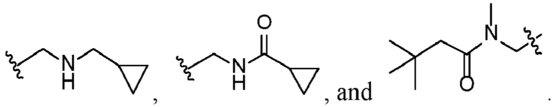

6. The compound, or the deuterated compound thereof, or the stereoisomer thereof, or the pharmaceutically acceptable salt thereof according to claim 1, wherein
R³ is selected from the group consisting of -C₀₋₂ alkylene-(3- to 10-membered carbocyclyl), -C₀₋₂ alkylene-(4- to 10-membered heterocycloalkyl), -C₀₋₂ alkylene-(6- to 10-membered aromatic ring), and -C₀₋₂ alkylene-(5- to 10-membered heteroaromatic ring); wherein the alkylene, carbocyclyl, heterocycloalkyl, aromatic ring, or heteroaromatic ring is optionally further substituted by one, two, three or four independent R³¹;
each R³¹ is independently selected from the group consisting of hydrogen, halogen, cyano, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen; or two independent R³¹, together with the atom to which they are linked directly, form 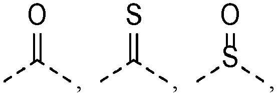 or 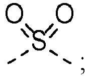
R⁴ is selected from the group consisting of hydrogen, -C₁₋₆ alkyl, -C₂₋₆ alkenyl, -C₂₋₆ alkynyl, -C₁₋₆ alkyl substituted with halogen, -C₂₋₆ alkenyl substituted with halogen, and -C₂₋₆ alkynyl substituted with halogen.

7. The compound, or the deuterated compound thereof, or the stereoisomer thereof, or the pharmaceutically acceptable salt thereof according to claim 6, wherein
R³ is selected from the group consisting of 3-membered carbocyclyl, 4-membered carbocyclyl, 5-membered carbocyclyl, 6-membered carbocyclyl, 7-membered carbocyclyl, 8-membered carbocyclyl, 9-membered carbocyclyl, 10-membered carbocyclyl, 4-membered heterocycloalkyl, 5-membered heterocycloalkyl, 6-membered heterocycloalkyl, 7-membered heterocycloalkyl, 8-membered heterocycloalkyl, 9-membered heterocycloalkyl, 10-membered heterocycloalkyl, 6-membered aromatic ring, 10-membered aromatic ring, 5-membered heteroaromatic ring, 6-membered heteroaromatic ring, 7-membered heteroaromatic ring, 8-membered heteroaromatic ring, 9-membered heteroaromatic ring, and 8-membered heteroaromatic ring; wherein the carbocyclyl, heterocycloalkyl, aromatic ring, or heteroaromatic ring is optionally further substituted by one, two, three or four independent R³¹;
R⁴ is selected from the group consisting of hydrogen and -C₁₋₆ alkyl.

8. The compound, or the deuterated compound thereof, or the stereoisomer thereof, or the pharmaceutically acceptable salt thereof according to claim 7, wherein R³ is selected from the group consisting of 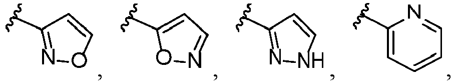 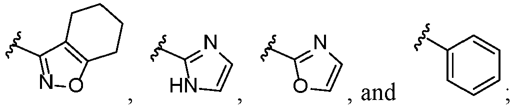 wherein the rings from which R³ is selected is optionally further substituted by one, two, three or four independent R³¹.

9. The compound, or the deuterated compound thereof, or the stereoisomer thereof, or the pharmaceutically acceptable salt thereof according to claim 8, wherein R³ is selected from the group consisting of

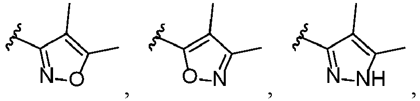

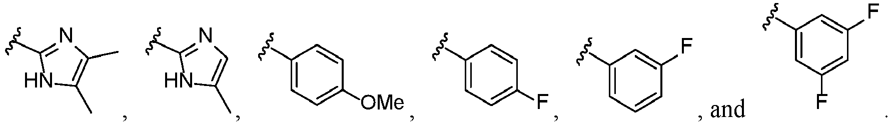

10. The compound, or the deuterated compound thereof, or the stereoisomer thereof, or the pharmaceutically acceptable salt thereof according to any one of claims 1 to 9, wherein
X is NR^{X1};
R^{X1} is selected from the group consisting of -C₁₋₆ alkyl, -C₂₋₆ alkenyl, -C₂₋₆ alkynyl, -C₁₋₆ alkyl substituted with halogen, -C₂₋₆ alkenyl substituted with halogen, -C₂₋₆ alkynyl substituted with halogen, -C₀₋₂ alkyl-(3- to 10-membered carbocyclyl), -C₀₋₂ alkyl-(4- to 10-membered heterocycloalkyl), -C₀₋₂ alkyl-(6- to 10-membered aromatic ring), and -C₀₋₂ alkyl-(5- to 10-membered heteroaromatic ring).

11. The compound, or the deuterated compound thereof, or the stereoisomer thereof, or the pharmaceutically acceptable salt thereof according to claim 10, wherein
R^{X1} is selected from the group consisting of -C₁₋₆ alkyl, -C₂₋₆ alkenyl, -C₂₋₆ alkynyl, 3-membered carbocyclyl, 4-membered carbocyclyl, 5-membered carbocyclyl, 6-membered carbocyclyl, 7-membered carbocyclyl, 8-membered carbocyclyl, 4-membered heterocycloalkyl, 5-membered heterocycloalkyl, 6-membered heterocycloalkyl, 7-membered heterocycloalkyl, 8-membered heterocycloalkyl, a benzene ring, a 5-membered heteroaromatic ring, and a 6-membered heteroaromatic ring.

12. The compound, or the deuterated compound thereof, or the stereoisomer thereof, or the pharmaceutically acceptable salt thereof according to claim 11, wherein

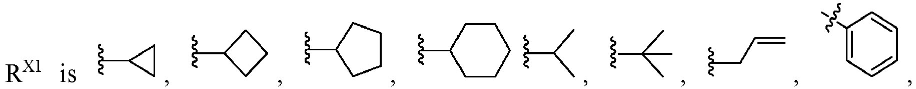

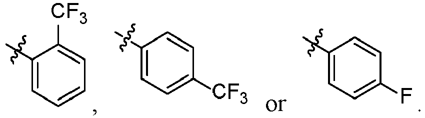

13. The compound, or the deuterated compound thereof, or the stereoisomer thereof, or the pharmaceutically acceptable salt thereof according to any one of claims 1 to 12, wherein the compound of formula I is represented by formula IIa or formula IIb: 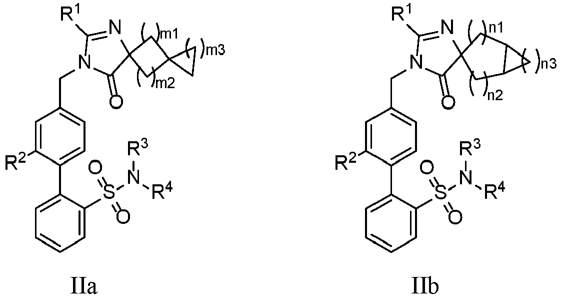
wherein m1 and m2 are each independently 0, 1, 2 or 3; and m3 is 1, 2, 3, 4 or 5;
n1 and n2 are each independently 0, 1, 2 or 3; and n3 is 1, 2, 3, 4 or 5.

14. The compound, or the deuterated compound thereof, or the stereoisomer thereof, or the pharmaceutically acceptable salt thereof according to any one of claims 1 to 13, wherein the compound is:

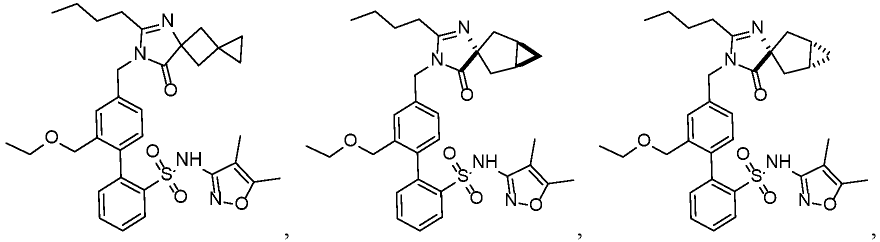

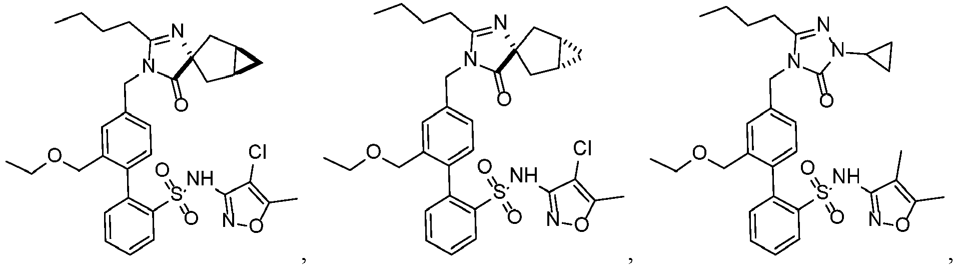

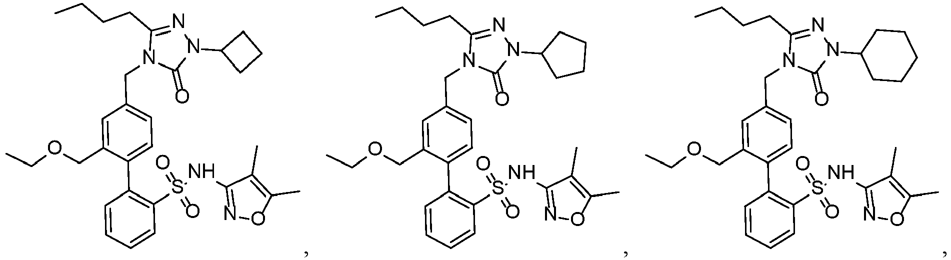

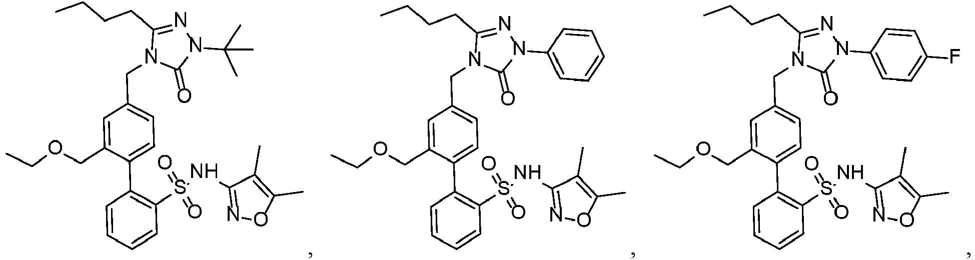

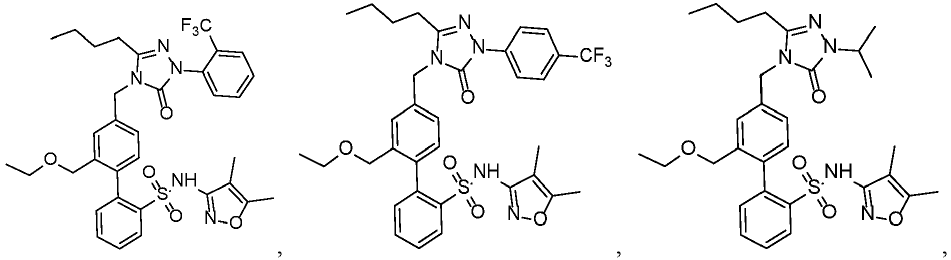

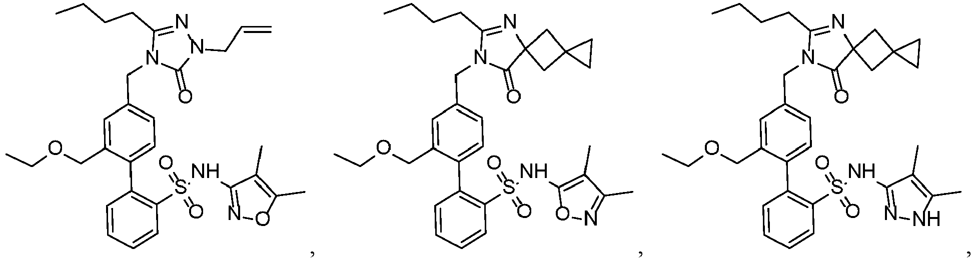

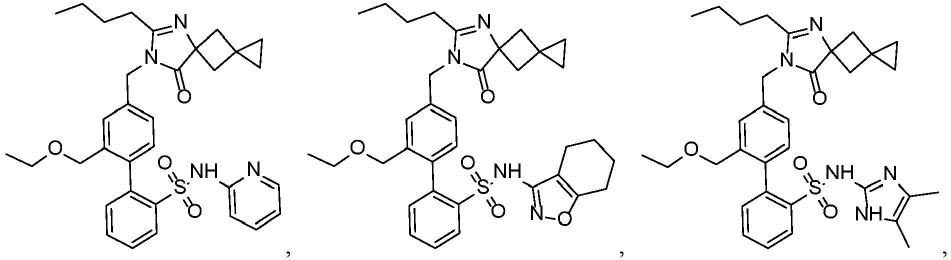

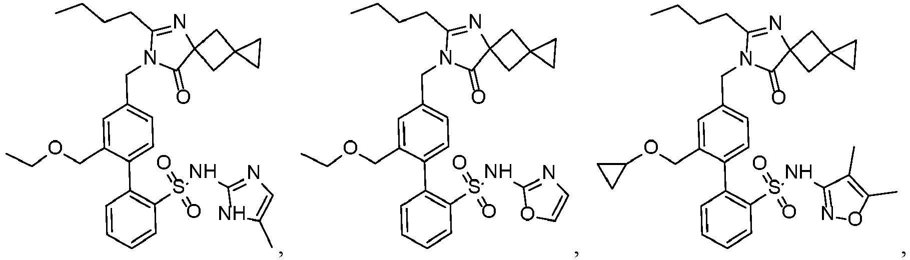

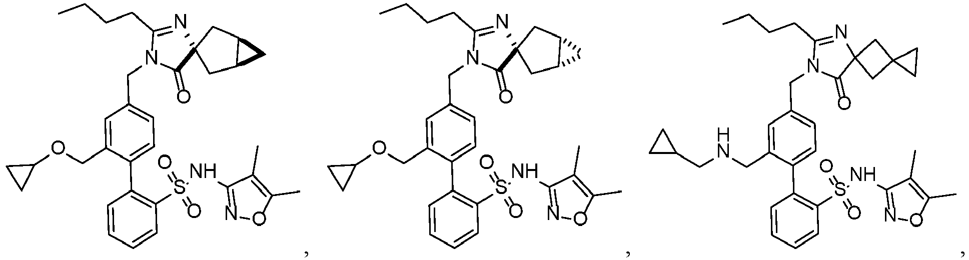

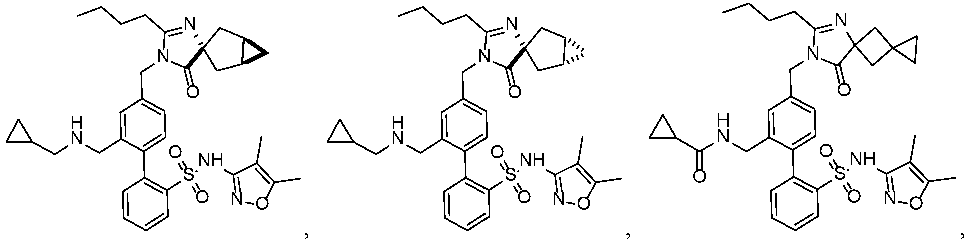

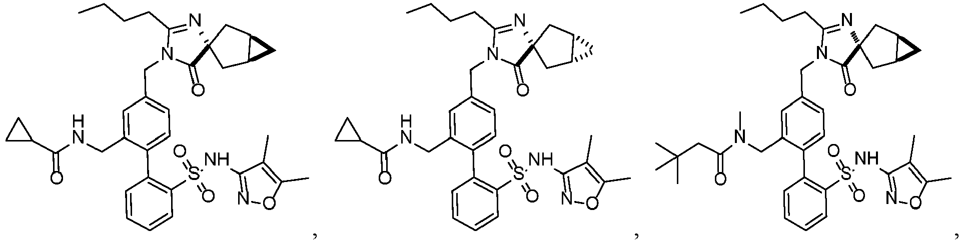

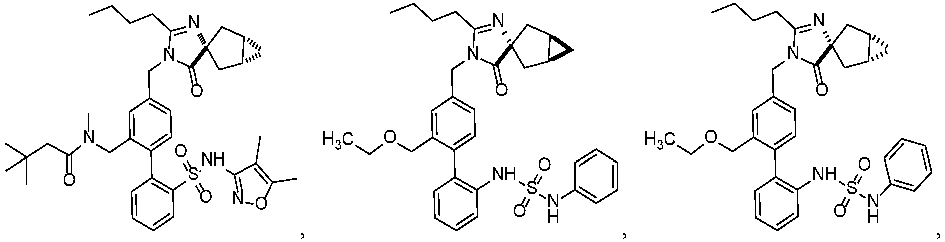

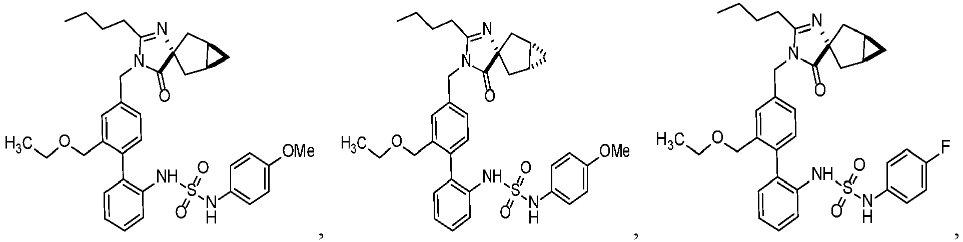

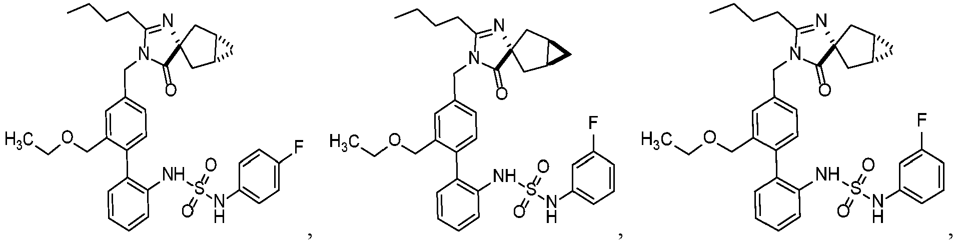

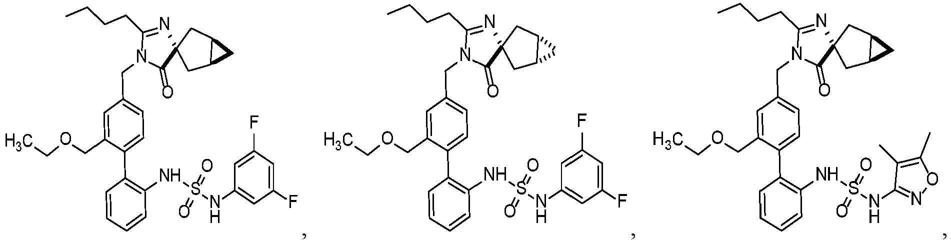

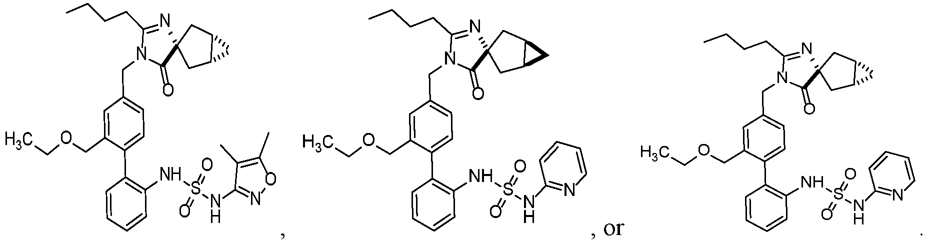

15. Use of the compound, or the deuterated compound thereof, or the stereoisomer thereof, or the pharmaceutically acceptable salt thereof according to any one of claims 1 to 14 in the manufacture of a medicament as dual angiotensin II receptor and endothelin receptor antagonist.

16. Use of the compound, or the deuterated compound thereof, or the stereoisomer thereof, or the pharmaceutically acceptable salt thereof according to any one of claims 1 to 14 in the manufacture of a medicament for treating cardiovascular and cerebrovascular diseases including hypertension, kidney diseases, and diseases associated with organ damage in relation to diabetes.

17. A pharmaceutical composition, comprising a formulation prepared from the compound, or the deuterated compound thereof, or the stereoisomer thereof, or the pharmaceutically acceptable salt thereof according to any one of claims 1 to 14.

18. The pharmaceutical composition according to claim 17, further comprising a pharmaceutically acceptable carrier, excipient, or vehicle.

19. A method for treating a disease or a condition associated with an angiotensin II receptor and an endothelin receptor, the method comprising administering, to a subject in need thereof, an effective amount of the compound, or the deuterated compound thereof, or the stereoisomer thereof, or the pharmaceutically acceptable salt thereof according to any one of claims 1 to 14, or the composition according to claim 17 or 18; preferably, the disease includes cardiovascular and cerebrovascular diseases including hypertension, kidney diseases, and diseases associated with organ damage in relation to diabetes.

20. A compound represented by formula IIIa or formula IIIb, or a deuterated compound thereof, or a stereoisomer thereof, or a pharmaceutically acceptable salt thereof: 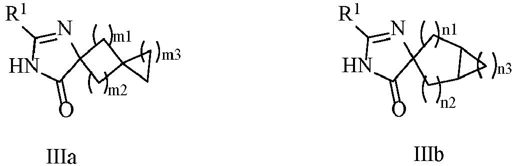 , wherein R¹ is -C₁₋₆ alkyl, -C₂₋₆ alkenyl, -C₂₋₆ alkynyl, -C₁₋₆ alkyl substituted with halogen, -C₂₋₆ alkenyl substituted with halogen or -C₂₋₆ alkynyl substituted with halogen, preferably n-butyl; m1 and m2 are each independently 0, 1, 2 or 3; and m3 is 1, 2, 3, 4 or 5; n1 and n2 are each independently 0, 1, 2 or 3; and n3 is 1, 2, 3, 4 or 5.
